(19) Europäisches Patentamt
European Patent Office
Office européen des brevets

(11) EP 3 067 411 A1

(12) EUROPÄISCHE PATENTANMELDUNG

(43) Veröffentlichungstag:
**14.09.2016 Patentblatt 2016/37**

(51) Int Cl.:
***C11D 3/386*** (2006.01) ***C12N 9/54*** (2006.01)

(21) Anmeldenummer: **16164786.2**

(22) Anmeldetag: **21.08.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.09.2011 DE 102011118027**

(62) Dokumentnummer(n) der früheren Anmeldung(en) nach Art. 76 EPÜ:
**12755969.8 / 2 756 064**

(71) Anmelder: **Henkel AG & Co. KGaA**
**40589 Düsseldorf (DE)**

(72) Erfinder:
- **SIEGERT, Petra**
  **42781 Haan (DE)**
- **EVERS, Stefan**
  **42781 Haan (DE)**
- **MERKEL, Marion**
  **51145 Köln (DE)**
- **MUßMANN, Nina**
  **47877 Willich (DE)**
- **HELLMUTH, Hendrik**
  **64297 Darmstadt (DE)**
- **O'CONNELL, Timothy**
  **40547 Düsseldorf (DE)**
- **MAURER, Karl-Heinz**
  **40699 Erkrath (DE)**
- **SCHWANEBERG, Ulrich**
  **4728 Kelmis-Hergenrath (BE)**
- **JAKOB, Felix**
  **41812 Erkelenz (DE)**
- **MARTINEZ-MOYA, Ronny**
  **50933 Köln (DE)**
- **LAUFS, Brian**
  **41363 Jüchen (DE)**
- **AYDEMIR, Ayhan**
  **40589 Düsseldorf (DE)**
- **WEBER, Thomas**
  **41541 Dormagen (DE)**

Bemerkungen:
Diese Anmeldung ist am 12.4.2016 als Teilanmeldung zu der unter INID-Code 62 erwähnten Anmeldung eingereicht worden.

(54) **VERFAHREN ZUR ANPASSUNG EINES HYDROLYTISCHEN ENZYMS AN EINE DAS HYDROLYTISCHE ENZYM STABILISIERENDE KOMPONENTE**

(57) Die Stabilisierung eines hydrolytischen Enzyms in einer flüssigen Zubereitung soll durch eine das hydrolytische Enzym stabilisierende Komponente verbessert werden. Dies gelingt durch ein Verfahren zur Anpassung eines hydrolytischen Enzyms an eine das hydrolytische Enzym stabilisierende Komponente, das die folgenden Verfahrensschritte umfasst:
a) Bereitstellen eines hydrolytischen Enzyms (Ausgangsenzym) und einer das hydrolytische Enzym stabilisierenden Komponente, die einen reversiblen Inhibitor des hydrolytischen Enzyms umfasst;
b) Verändern der Aminosäuresequenz des hydrolytischen Enzyms an mindestens einer Position durch Substitution, Deletion oder Insertion;
c) Bestimmen der relativen Aktivität des hydrolytischen Enzyms aus Schritt b) und der relativen Aktivität des Ausgangsenzyms in einer flüssigen Zubereitung;
d) Auswählen desjenigen hydrolytischen Enzyms, das eine verminderte relative Aktivität aufweist im Vergleich mit der relativen Aktivität des Ausgangsenzyms.

EP 3 067 411 A1

SEQUENCE LISTING

<110> Henkel AG & Co. KGaA

<120> Verfahren zur Anpassung eines hydrolytischen Enzyms an eine das hydrolytische Enzym stabilisierende Komponente

<130> H 09325-I (PT018970)

<150> DE 102011118027.7
<151> 2011-09-12

<160> 3

<170> PatentIn version 3.5

<210> 1
<211> 275
<212> PRT
<213> Bacillus amyloliquefaciens

<400> 1

```
Ala Gln Ser Val Pro Tyr Gly Val Ser Gln Ile Lys Ala Pro Ala Leu
1               5                   10                  15

His Ser Gln Gly Tyr Thr Gly Ser Asn Val Lys Val Ala Val Ile Asp
            20                  25                  30

Ser Gly Ile Asp Ser Ser His Pro Asp Leu Lys Val Ala Gly Gly Ala
        35                  40                  45

Ser Met Val Pro Ser Glu Thr Asn Pro Phe Gln Asp Asn Asn Ser His
    50                  55                  60

Gly Thr His Val Ala Gly Thr Val Ala Ala Leu Asn Asn Ser Ile Gly
65                  70                  75                  80

Val Leu Gly Val Ala Pro Ser Ala Ser Leu Tyr Ala Val Lys Val Leu
                85                  90                  95

Gly Ala Asp Gly Ser Gly Gln Tyr Ser Trp Ile Ile Asn Gly Ile Glu
            100                 105                 110

Trp Ala Ile Ala Asn Asn Met Asp Val Ile Asn Met Ser Leu Gly Gly
        115                 120                 125

Pro Ser Gly Ser Ala Ala Leu Lys Ala Ala Val Asp Lys Ala Val Ala
    130                 135                 140

Ser Gly Val Val Val Val Ala Ala Ala Gly Asn Glu Gly Thr Ser Gly
145                 150                 155                 160
```

```
Ser Ser Ser Thr Val Gly Tyr Pro Gly Lys Tyr Pro Ser Val Ile Ala
                165                 170                 175

Val Gly Ala Val Asp Ser Ser Asn Gln Arg Ala Ser Phe Ser Ser Val
            180                 185                 190

Gly Pro Glu Leu Asp Val Met Ala Pro Gly Val Ser Ile Gln Ser Thr
        195                 200                 205

Leu Pro Gly Asn Lys Tyr Gly Ala Tyr Asn Gly Thr Ser Met Ala Ser
    210                 215                 220

Pro His Val Ala Gly Ala Ala Ala Leu Ile Leu Ser Lys His Pro Asn
225                 230                 235                 240

Trp Thr Asn Thr Gln Val Arg Ser Ser Leu Glu Asn Thr Thr Thr Lys
                245                 250                 255

Leu Gly Asp Ser Phe Tyr Tyr Gly Lys Gly Leu Ile Asn Val Gln Ala
            260                 265                 270

Ala Ala Gln
        275


<210>  2
<211>  485
<212>  PRT
<213>  Bacillus sp.

<400>  2

His His Asn Gly Thr Asn Gly Thr Met Met Gln Tyr Phe Glu Trp Tyr
1               5                   10                  15

Leu Pro Asn Asp Gly Asn His Trp Asn Arg Leu Arg Ser Asp Ala Ser
            20                  25                  30

Asn Leu Lys Asp Lys Gly Ile Ser Ala Val Trp Ile Pro Pro Ala Trp
        35                  40                  45

Lys Gly Ala Ser Gln Asn Asp Val Gly Tyr Gly Ala Tyr Asp Leu Tyr
    50                  55                  60

Asp Leu Gly Glu Phe Asn Gln Lys Gly Thr Ile Arg Thr Lys Tyr Gly
65                  70                  75                  80

Thr Arg Asn Gln Leu Gln Ala Ala Val Asn Ala Leu Lys Ser Asn Gly
                85                  90                  95
```

```
Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Ser Gly Glu Tyr Thr Ile Glu Ala Trp Thr Lys Phe Asp
    130                 135                 140

Phe Pro Gly Arg Gly Asn Thr His Ser Asn Phe Lys Trp Arg Trp Tyr
145                 150                 155                 160

His Phe Asp Gly Val Asp Trp Asp Gln Ser Arg Lys Leu Asn Asn Arg
                165                 170                 175

Ile Tyr Lys Phe Arg Gly Asp Gly Lys Gly Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
                245                 250                 255

Thr Gly Lys Asn Met Phe Ala Val Ala Glu Phe Trp Lys Asn Asp Leu
            260                 265                 270

Gly Ala Ile Glu Asn Tyr Leu Asn Lys Thr Asn Trp Asn His Ser Val
        275                 280                 285

Phe Asp Val Pro Leu His Tyr Asn Leu Tyr Asn Ala Ser Lys Ser Gly
    290                 295                 300

Gly Asn Tyr Asp Met Arg Gln Ile Phe Asn Gly Thr Val Val Gln Arg
305                 310                 315                 320

His Pro Met His Ala Val Thr Phe Val Asp Asn His Asp Ser Gln Pro
                325                 330                 335

Glu Glu Ala Leu Glu Ser Phe Val Glu Glu Trp Phe Lys Pro Leu Ala
            340                 345                 350
```

```
Tyr Ala Leu Thr Leu Thr Arg Glu Gln Gly Tyr Pro Ser Val Phe Tyr
        355                 360                 365

Gly Asp Tyr Tyr Gly Ile Pro Thr His Gly Val Pro Ala Met Lys Ser
    370                 375                 380

Lys Ile Asp Pro Ile Leu Glu Ala Arg Gln Lys Tyr Ala Tyr Gly Arg
385                 390                 395                 400

Gln Asn Asp Tyr Leu Asp His His Asn Ile Ile Gly Trp Thr Arg Glu
                405                 410                 415

Gly Asn Thr Ala His Pro Asn Ser Gly Leu Ala Thr Ile Met Ser Asp
            420                 425                 430

Gly Ala Gly Gly Asn Lys Trp Met Phe Val Gly Arg Asn Lys Ala Gly
        435                 440                 445

Gln Val Trp Thr Asp Ile Thr Gly Asn Arg Ala Gly Thr Val Thr Ile
    450                 455                 460

Asn Ala Asp Gly Trp Gly Asn Phe Ser Val Asn Gly Gly Ser Val Ser
465                 470                 475                 480

Ile Trp Val Asn Lys
                485


<210>  3
<211>  269
<212>  PRT
<213>  Bacillus lentus

<400>  3

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80
```

```
Gly Val Ala Pro Ser Ala Glu Leu Tyr Ala Val Lys Val Leu Gly Ala
                85                  90                  95

Asp Gly Glu Gly Ala Ile Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Ser Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265
```

## Beschreibung

Die Erfindung liegt auf dem Gebiet der Enzymtechnologie. Die Erfindung betrifft insbesondere ein Verfahren, in dem ein hydrolytisches Enzym, insbesondere eine Protease, an einen Enzymstabilisator angepasst und folglich für diesen optimiert wird. Ferner betrifft die Erfindung hydrolytische Enzyme, die mit einem derartigen Verfahren erhältlich sind.

Probleme betreffend die Lagerstabilität enzymhaltiger Zubereitungen, beispielsweise von Enzympräparationen oder von Wasch-, Reinigungs- oder Desinfektionsmitteln, sind aus dem Stand der Technik bekannt. Besonders ausgeprägt ist diese Problematik bei flüssigen Enzympräparationen beziehungsweise flüssigen enzymhaltigen Tensidzubereitungen, beispielsweise flüssigen Wasch- oder Reinigungsmitteln. Sie büßen bereits nach kurzer Zeit ein erhebliches Maß an enzymatischer, insbesondere hydrolytischer und besonders an proteolytischer, Aktivität ein. Die Tensidzubereitung, beispielsweise das Wasch-, Reinigungs- oder Desinfektionsmittel, zeigt dann keine optimale Reinigungsleistung mehr. Ein Ziel bei der Entwicklung enzymhaltiger Zubereitungen besteht daher darin, die enthaltenen Enzyme zu stabilisieren und sie besonders während der Lagerung und/oder während der Anwendung der Zubereitung vor Denaturierung, Spaltung und/oder sonstigem Abbau zu schützen. Diesbezüglich sind besonders hydrolytische Enzyme und insbesondere Proteasen, aber beispielsweise auch Amylasen, von Interesse. Hierfür können den Zubereitungen chemische Verbindungen zugesetzt werden, die insbesondere Proteasen reversibel inhibieren und somit insgesamt als Stabilisatoren für die Proteasen und andere enthaltene Enzyme wirken. Es muss sich dabei um reversible Inhibitoren handeln, da die Enzymaktivität nur vorübergehend, insbesondere während der Lagerung, nicht aber mehr während des Reinigungsprozesses unterbunden werden soll.

Unter bereits in vergleichsweise niedriger Konzentration in Tensidzubereitungen wirksamen Enzymstabilisatoren nehmen Borsäure und Borsäurederivate eine herausragende Stellung ein. Beispielsweise offenbart die internationale Patentanmeldung WO 96/21716 A1, dass als Proteaseinhibitoren wirkende Bor- und Boronsäurederivate geeignet sind, Enzyme in flüssigen Zubereitungen, darunter in Wasch- und Reinigungsmitteln, zu stabilisieren. Eine Auswahl von Boronsäure-Derivaten als Stabilisatoren ist beispielsweise offenbart in der internationalen Patentanmeldung WO 96/41859 A1. Aus WO 92/19707 A1 und EP 478050 A1 gehen meta- bzw. parasubstituierte Phenylboronsäuren als Enzymstabilisatoren hervor. Komplexe von Borsäuren und Borsäure-Derivaten mit aromatischen Verbindungen als Enzymstabilisatoren in flüssigen Detergenszusammensetzungen sind offenbart in EP 511456 A1.

Allerdings weisen Borsäuren und Borsäurederivate den Nachteil auf, dass sie mit anderen Inhaltsstoffen einer Tensidzubereitung, insbesondere Wasch-, Reinigungs- oder Desinfektionsmittelinhaltsstoffen, unerwünschte Nebenprodukte bilden, so dass diese in den betreffenden Mitteln nicht mehr für den erwünschten Reinigungszweck zur Verfügung stehen oder sogar als Verunreinigung zurückbleiben, beispielsweise auf dem Waschgut. Ferner werden Borsäuren bzw. Borate unter Umweltaspekten zunehmend als nachteilig betrachtet.

Aus dem Stand der Technik sind zwar auch Borsäure-freie oder borfreie Verbindungen bekannt, die hydrolytische Enzyme reversibel inhibieren und somit stabilisieren können. Diese weisen jedoch häufig den Nachteil auf, dass sie keine zufrieden stellende Enzymstabilisierung in einer Zubereitung des Enzyms bewirken, insbesondere nicht in flüssigen Tensidzubereitungen wie beispielsweise in Wasch-, Reinigungs- oder Desinfektionsmitteln. Um eine ausreichende Stabilisierung zu bewirken, müssten derartige Verbindungen häufig in einer so großen Menge eingesetzt werden, was den Einsatz oftmals nicht möglich macht, da diese Mengen nicht zufrieden stellend in die Enzymzubereitung eingearbeitet werden können und/oder sich deren Einarbeitung wirtschaftlich nicht rentabel gestaltet.

Der vorliegenden Erfindung liegt die Aufgabe zugrunde, ein hydrolytisches Enzym an eine das hydrolytische Enzym stabilisierende Komponente, insbesondere einen reversiblen Inhibitor, anzupassen und folglich dessen Stabilisierung durch diese Komponente zu verbessern. Vorzugsweise sollte die das Enzym stabilisierende Komponente eine möglichst geringe Menge an Borsäure oder Bor-haltigen Verbindungen umfassen oder vorzugsweise frei von Borsäure und/oder Bor sein.

Gegenstand der Erfindung ist ein Verfahren zur Anpassung eines hydrolytischen Enzyms an eine das hydrolytische Enzym stabilisierende Komponente umfassend die Verfahrensschritte

a) Bereitstellen eines hydrolytischen Enzyms (Ausgangsenzym) und einer das hydrolytische Enzym stabilisierenden Komponente, die einen reversiblen Inhibitor des hydrolytischen Enzyms umfasst;
b) Verändern der Aminosäuresequenz des hydrolytischen Enzyms an mindestens einer Position durch Substitution, Deletion oder Insertion;
c) Bestimmen der relativen Aktivität des hydrolytischen Enzyms aus Schritt b) und der relativen Aktivität des Ausgangsenzyms in einer flüssigen Zubereitung;
d) Auswählen desjenigen hydrolytischen Enzyms, das eine verminderte relative Aktivität aufweist im Vergleich mit der relativen Aktivität des Ausgangsenzyms.

Ein erfindungsgemäßes Verfahren kann ferner noch den folgenden Verfahrensschritt e) umfassen:

e) optionales Wiederholen der Verfahrensschritte b) bis d), bis das hydrolytische Enzym eine verminderte relative Aktivität aufweist im Vergleich mit der relativen Aktivität des Ausgangsenzyms.

Im Rahmen der vorliegenden Erfindung wird folglich ein außergewöhnlicher Ansatz verfolgt. Entgegen der im Stand der Technik etablierten Vorgehensweise, für ein gegebenes hydrolytisches Enzym eine Komponente aufzufinden, welche dieses Enzym stabilisiert, wird erfindungsgemäß der umgekehrte Weg beschritten, nämlich dass das hydrolytische Enzym verändert und dadurch an die das hydrolytische Enzym stabilisierende Komponente (Enzymstabilisator), insbesondere an den reversiblen Inhibitor, angepasst wird, so dass eine verbesserte Stabilisierung des veränderten hydrolytischen Enzyms durch die das hydrolytische Enzym stabilisierende Komponente erreicht wird. Es wird nicht der passende Stabilisator für ein Enzym gesucht, sondern das Enzym für dessen Stabilisierung durch den gegebenen Stabilisator optimiert. Es wurde gefunden, dass es vorteilhaft ist, das hydrolytische Enzym an die das hydrolytische Enzym stabilisierende Komponente anzupassen und nicht umgekehrt. Auf diese Art und Weise können beispielsweise in kurzer Zeit vorteilhafte Kombinationen von Enzym und stabilisierender Verbindung identifiziert werden. In bevorzugten Ausgestaltungen resultieren derartige Kombinationen in einer verbesserten Enzyminhibierung und/oder Enzymstabilisierung durch die stabilisierende Verbindung, insbesondere in einem flüssigen Wasch-, Reinigungs- oder Desinfektionsmittel. Hierdurch eröffnet sich die Möglichkeit, in flüssigen Zubereitungen, insbesondere flüssigen Tensidzubereitungen, weniger Borsäure-haltige und/oder Bor-haltige Verbindungen als Enzymstabilisatoren einzusetzen. Insbesondere ist es in besonders bevorzugten Ausgestaltungen möglich, in einer derartigen flüssigen Zubereitung auf Borsäure als Enzymstabilisator teilweise oder vorzugsweise vollständig zu verzichten, so dass die Zubereitung frei von Borsäure sein kann. In weiteren besonders vorteilhaften Ausgestaltungen kann eine solche Zubereitung idealerweise frei von Bor sein.

Vorzugsweise weisen erfindungsgemäß angepasste Kombinationen von Enzymstabilisator und hydrolytischem Enzym ferner den Vorteil auf, dass der Enzymstabilisator bereits in geringen bis sehr geringen Konzentrationen seine stabilisierende Wirkung entfaltet. Weiter kann er so ausgewählt werden, dass er vorzugsweise über eine gute Wasserlöslichkeit verfügt. Daher kann er - zusammen mit dem durch ihn stabilisierten Enzym - in eine wässrige flüssige Zubereitung, insbesondere eine Tensidzubereitung und hierunter vorzugsweise in ein flüssiges Wasch-, Reinigungs- oder Desinfektionsmittel, einfach eingearbeitet werden. Vorzugsweise wird ferner ein Ausfällen während der Lagerung vermindert bzw. ganz vermieden.

Ein hydrolytisches Enzym ist eine Hydrolase (E.C. 3.X.X.X) und damit ein Enzym, das Ester, Ether, Peptide, Glykoside, Säureanhydride oder C-C-Bindungen in reversibler Reaktion hydrolytisch spaltet. Das hydrolytische Enzym katalysiert daher die hydrolytische Spaltung von Stoffen gemäß $A\text{-}B + H_2O \leftrightarrow AH + B\text{-}OH$. Hydrolasen bilden die dritte Hauptklasse der EC-Klassifikation der Enzyme. Die EC-Nummern (engl. "Enzyme Commission numbers") bilden ein numerisches Klassifikationssystem für Enzyme. Jede EC-Nummer besteht aus vier durch Punkte voneinander getrennten Zahlen, wobei die erste Ziffer eine der sechs Enzymhauptklassen bezeichnet und Hydrolasen mit E.C. 3.X.X.X entsprechend die dritte Hauptklasse darstellen. Ihre Vertreter sind Proteasen, Peptidasen, Nukleasen, Phosphatasen, Glykosidasen und Esterasen. Besonders bevorzugt ist das hydrolytische Enzym eine Protease.

In Verfahrensschritt a) wird ein hydrolytisches Enzym sowie eine das hydrolytische Enzym stabilisierende Komponente bereitgestellt, die einen reversiblen Inhibitor des hydrolytischen Enzyms umfasst. Ein reversibler Inhibitor des hydrolytischen Enzyms ist diesbezüglich jede Verbindung, die zu einer nichtkovalenten Wechselwirkung mit dem hydrolytischen Enzym reversibel befähigt ist und die katalytische Aktivität des hydrolytischen Enzyms aufgrund ihrer Wechselwirkung mit dem Enzym vermindert. Die Stabilisierung des hydrolytischen Enzyms erfolgt daher auf Grund seiner Hemmung durch den reversiblen Inhibitor, vorzugsweise in einer wässrigen Umgebung. Wasser selbst ist folglich keine das hydrolytische Enzym stabilisierende Komponente im Rahmen der vorliegenden Erfindung. Vorzugsweise weist der reversible Inhibitor des hydrolytischen Enzyms eine molare Masse von mindestens 20 g/mol und zunehmend bevorzugt von mindestens 25, 30, 35, 40, 45, 50, 55, 60, 65, 70 g/mol auf. Weiter bevorzugt handelt es sich diesbezüglich um eine organisch chemische Verbindung.

Vorzugsweise weist der reversible Inhibitor bezüglich des hydrolytischen Enzyms in Verfahrensschritt a) eine Inhibitionskonstante (Ki) von 0,01 bis 500 mM, vorzugsweise von 0,01 bis 100 mM, besonders bevorzugt von 0,01 bis 1 mM oder von 10 bis 100 mM auf.

Die Inhibitionskonstante $K_i$ kann auf folgende Weise ermittelt werden:

Für die Charakterisierung eines reversiblen Inhibitors der enzymatischen Aktivität ist die Inhibitionskonstante $K_i$ eine charakteristische und entscheidende Größe. $K_i$ beschreibt das Gleichgewicht zwischen Enzym, Inhibitor und Enzym-Inhibitor Komplex für eine reversible Bindung. Der Enzym-Inhibitor Komplex ist dabei katalytisch nicht aktiv und inhibiert die Reaktion durch Herabsetzung der Konzentration von freiem Enzym, das noch zur Bindung von Substrat zur Verfügung steht. Der $K_i$ ist dementsprechend definiert als:

$$K_i = [I] \times [E]/[EI]$$

Darin bedeuten [E], [I] und [EI] die jeweiligen molaren Gleichgewichtskonzentrationen von Enzym (E), Inhibitor (I) und dem Enzym-Inhibitor-Komplex (EI). Dieser Definition entsprechend ist eine Substanz mit einem kleinen Ki unter den jeweiligen Testbedingungen ein guter Inhibitor.

Die Bestimmung des $K_i$ erfolgt auf der Grundlage des Aktivitätstests der Protease in Anwesenheit des entsprechenden Inhibitors. Über die im Stand der Technik etablierte und dem Fachmann bekannte Michaelis-Menten-Kinetik (Leonor Michaelis, Maud Menten (1913). Die Kinetik der Invertinwirkung, Biochem. Z. 49:333-369) werden die enzymatischen Parameter $K_m$, und $k_{cat}$ in Anwesenheit verschiedener Konzentrationen des Inhibitors bestimmt. Für eine Michaelis-Menten-Kinetik gilt vereinfacht:

$$E + S \underset{k_{-1}}{\overset{k_1}{\rightleftharpoons}} ES \xrightarrow{k_2} E + P$$

Hierin bedeuten: E: Enzym
S: Substrat
ES: Enzym-Substrat-Komplex
P: Produkt
$k_1, k_{-1}, k_2$: Geschwindigkeitskonstanten

Hierin ist $k_2$ ein Maß der maximalen Reaktionsgeschwindigkeit bei Substratsättigung (Vmax), auch Wechselzahl, molekulare Aktivität, "turnover number" oder kcat genannt (kcat = Vmax/ [Eo], wobei [Eo] die Ausgangskonzentration des Enzyms ist). Die Michaeliskonstante (d.h. die Substratkonzentration, die bei der Halbsättigung vorliegt, bei der die Umsatzgeschwindigkeit also v = Vmax/2 beträgt), ergibt sich zu
$K_m = k_{-1} / k_1$ (Michaelis-Menten-Fall, gegeben wenn $k_2 << k_1$) oder bzw. allgemeiner zu
$K_m = k_{-1} + k_{2\,/\,k1}$ (Briggs-Haldane-Situation, gegeben für den Fall, dass $k_2$ gegenüber $k_1$ nicht vernachlässigt werden kann).

Die Sättigungsfunktion eines "Michaelis-Menten Enzyms" ergibt sich unter Verwendung der Parameter Km und Vmax wie folgt:

$$v = \frac{v_{max} \cdot [S]}{K_m + [S]}$$

Hierin bedeutet:

v: Bildungsgeschwindigkeit von P (v = "Geschwindigkeit") [mol I-1 s-1]
vmax: maximale Geschwindigkeit [mol I-1 s-1]
Km: Michaelis-Menten-Konstante [mol I-1]
[S]: Substratkonzentration [mol I-1]

Durch die Bestimmung der Anfangskatalyse-Rate ($v_{Anf.}$) - für Proteasen der Anfangshydrolyse-Rate - bei verschiedenen Substratkonzentrationen [S] und Einpassung der experimentellen Daten in nachstehende Gleichung 1 wird die Inhibitionskonstante $K_i$ erhalten.

Gleichung 1: $v_{Anf.} = k_{cat} \times [S] \times E_0 / (K_m \times (1 + [I]/K_i) + S)$

Hierin steht [I] wiederum für die Inhibitor-Konzentration.

Alternativ kann $K_i$ unter Verwendung der Cheng-Prusoff-Gleichung (Gleichung 2, Cheng Y., Prusoff W.H. (1973) Biochem.Pharmacol. 22, 3099-3108) über den $IC_{50}$-Wert bestimmt werden. Die Bestimmung des $IC_{50}$-Werts erfolgt über die Bestimmung der katalytischen Aktivität an einem Substrat in Anwesenheit verschiedener Konzentrationen des Inhibitors und der Anpassung der experimentellen Daten an eine sigmoidale Dosis-Wirkungs-Gleichung mit variabler

Steigung (Pseudo-Hill-Steigungen). Es handelt sich dabei um die Inhibitor-Konzentration, die nötig ist, um eine 50%ige Inhibition zu erreichen.

$K_i$ ergibt sich damit aus folgender Gleichung 2:

$$\text{Gleichung 2:} \quad K_i = IC_{50}/(1 + [S]/K_d)$$

Darin bedeuten [S] die Substratkonzentration im Test und $K_d$ die Dissoziationskonstante für das Substrat, die bei der $IC_{50}$-Konzentration des Inhibitors als identisch mit $K_m$ für das Substrat gesetzt werden kann.

Die auf diese Weise bestimmbaren $K_i$-Werte kennzeichnen den reversiblen Inhibitor in Bezug auf das eingesetzte Enzym, also beispielsweise den reversiblen Proteaseinhibitor in Bezug auf die eingesetzte Protease.

Die das hydrolytische Enzym stabilisierende Komponente kann vollständig aus dem reversiblen Inhibitor bestehen, so dass die das hydrolytische Enzym stabilisierende Komponente (Enzymstabilisator) der reversible Inhibitor ist. Diese Ausgestaltung ist besonders bevorzugt. Alternativ kann die das hydrolytische Enzym stabilisierende Komponente weitere Verbindungen umfassen, so dass der jeweilige reversible Inhibitor ein Teil der das hydrolytische Enzym stabilisierenden Komponente ist. Insbesondere kann die das hydrolytische Enzym stabilisierende Komponente Kombinationen unterschiedlicher reversibler Inhibitoren umfassen.

Vorzugsweise bewirkt die das hydrolytische Enzym stabilisierende Komponente (in Ergänzung zu der inhibitorischen Wirkung des reversiblen Inhibitors) ferner bereits eine Lagerstabilität des hydrolytischen Enzyms in einer wässrigen Umgebung auf. Durch ein erfindungsgemäßes Verfahren wird die Lagerstabilität dann vorzugsweise weiter verbessert, indem das hydrolytische Enzym an die das hydrolytische Enzym stabilisierende Komponente, insbesondere an den reversiblen Inhibitor, angepasst wird.

Eine Lagerstabilität liegt vor, wenn die Anwesenheit der das hydrolytische Enzym stabilisierenden Komponente bewirkt, dass eine wasserhaltige flüssige Zubereitung umfassend hydrolytisches Enzym und hydrolytisches Enzym stabilisierende Komponente (stabilisierte Zubereitung) nach einer Lagerung eine höhere enzymatische Restaktivität des hydrolytischen Enzyms aufweist im Vergleich zu einer Kontrollzubereitung, die sich nur durch die Abwesenheit von der das hydrolytische Enzym stabilisierenden Komponente von der stabilisierten Zubereitung unterscheidet (Kontrolle). Diesbezüglich ist die das hydrolytische Enzym stabilisierende Komponente in einer Menge von 0,002 bis 0,35 Gew.-% in der stabilisierten Zubereitung enthalten. Nach Lagerung weist diese Zubereitung daher eine höhere Restaktivität des hydrolytischen Enzyms auf im Vergleich zur Kontrolle, wobei die stabilisierte Zubereitung und die Kontrolle die gleiche enzymatische Ausgangsaktivität bei Lagerbeginn aufweisen, beide Zubereitungen auf die gleiche Art und Weise behandelt werden, insbesondere betreffend die Bedingungen der Lagerung und die Bestimmung der Enzymaktivität. Die Lagerung erfolgt vorzugsweise für mindestens 48 Stunden bei einer Temperatur von 20°C, 25°C oder 30°C. Zunehmend bevorzugt erfolgt die Lagerung für mindestens 72 Stunden, 5 Tage, 1 Woche, 2 Wochen, 3 Wochen, 4 Wochen, 5 Wochen, 6 Wochen, 7 Wochen oder 8 Wochen bei einer Temperatur von 20°C, 25°C oder 30°C über die gesamte Lagerdauer.

Besonders bevorzugt wird das Vorliegen einer Lagerstabilität ermittelt

i. für eine Protease-haltige flüssige Zubereitung unter Verwendung dieser Protease-haltigen flüssigen Zubereitung, die für mindestens 48 Stunden bei einer Temperatur von 30°C gelagert wird und deren proteolytische Restaktivität bestimmt wird über die Freisetzung des Chromophors para-Nitroanilin (pNA) aus dem Substrat suc-AAPF-pNA wie nachstehend angegeben; und/oder

ii. für eine Amylase-haltige flüssige Zubereitung unter Verwendung dieser Amylase-haltigen, vorzugsweise proteasefreien, flüssigen Zubereitung, die für mindestens 48 Stunden bei einer Temperatur von 30°C gelagert wird, und deren amylolytische Restaktivität bestimmt wird wie nachstehend angegeben.

Zur Verdeutlichung wird an dieser Stelle angemerkt, dass erfindungsgemäß in Verfahrensschritt a) mindestens ein reversibler Inhibitor des hydrolytischen Enzyms vorliegt, dessen inhibitorische Wirkung vorzugsweise - aber nicht zwingend - bereits eine Lagerstabilität des hydrolytischen Enzyms bewirkt, wobei die Lagerstabilität vorzugsweise wie vorstehend beschrieben ermittelt wird. Zur bloßen Feststellung einer inhibitorischen Wirkung einer Verbindung auf ein hydrolytisches Enzym ist das Vorliegen einer Lagerstabilität allerdings nicht notwendig - diesbezüglich ist die Bestimmung der Aktivität des hydrolytischen Enzyms in An- und Abwesenheit der Verbindung entscheidend. Es ist demnach zwischen inhibitorischer Wirkung und Lagerstabilität zu differenzieren. Ein reversibler Inhibitor weist eine inhibitorische Wirkung auf - diese bedingt vorzugsweise, aber nicht zwingend, eine Lagerstabilität.

Zur Anpassung des hydrolytischen Enzyms an die das hydrolytische Enzym stabilisierende Komponente, insbesondere an den reversiblen Inhibitor, wird in Verfahrensschritt b) die Aminosäuresequenz des hydrolytischen Enzyms an mindestens einer Position durch Substitution, Deletion oder Insertion verändert. Es können ferner auch mehrere

Substitutionen, Deletionen oder Insertionen vorgenommen werden. Auch beliebige Kombinationen von einer oder mehreren Substitutionen, Deletionen oder Insertionen können erfindungsgemäß vorgenommen werden. Das Einbringen derartiger Mutationen in eine Aminosäuresequenz ist dem Fachmann auf dem Gebiet der Enzymtechnologie geläufig und erfolgt vorzugsweise durch Veränderung der für das hydrolytische Enzym codierenden Nukleinsäuresequenz. Das veränderte Enzym selbst wird dann durch Expression dieser Nukleinsäuresequenz in einem geeigneten Wirt erhalten und gegebenenfalls anschließend aufgereinigt. Im Falle einer Insertion wird die zu inserierende Aminosäure nach der jeweiligen Aminosäure (d.h. C-terminal) eingefügt.

In Verfahrensschritt c) wird die relative Aktivität des in Verfahrensschritt b) veränderten hydrolytischen Enzyms sowie des Ausgangsenzyms bestimmt. Für die Bestimmung der relativen Aktivität ist es notwendig, die Aktivitätsbestimmung für ein Enzym sowohl in An- als auch in Abwesenheit von der das hydrolytische Enzym stabilisierenden Komponente in einer flüssigen Zubereitung durchzuführen, insbesondere in einer wasserhaltigen flüssigen Zubereitung. Vorzugsweise ist die flüssigen Zubereitung zusammengesetzt wie folgt (alle Angaben in GewichtsProzent): 0,3-0,5% Xanthan, 0,2-0,4% Anti-Schaummittel, 6-7% Glycerin, 0,3-0,5% Ethanol, 4-7% FAEOS (Fettalkoholethersulfat), 24-28% nichtionische Tenside, 1-2% Natriumcitrat (Dihydrat), 2-4% Soda, 14-16% Kokosnuss-Fettsäuren, 0,5% HEDP (1-Hydroxyethan-(1,1-diphosphonsäure)), 0-0,4% PVP (Polyvinylpyrrolidon), 0-0,05% optischer Aufheller, 0-0,001% Farbstoff, Rest demineralisiertes Wasser. Eine derartige Zubereitung oder eine 50%ige wässrige Lösung derselben wird besonders bevorzugt für die Bestimmung der relativen Aktivität verwendet.

Die Bestimmung der Enzymaktivität erfolgt - abgestimmt auf den jeweiligen Enzymtyp - in fachüblicher Art und Weise. Methoden zur Aktivitätsbestimmung sind dem Fachmann auf dem Gebiet der Enzymtechnologie geläufig und werden von ihm routinemäßig angewendet. Verfahren zur Bestimmung der Proteaseaktivität sind beispielsweise offenbart in Tenside, Band 7 (1970), S. 125-132. Vorzugsweise wird die proteolytische Aktivität über die Freisetzung des Chromophors para-Nitroanilin (pNA) aus dem Substrat suc-L-Ala-L-Ala-L-Pro-L-Phe-p-Nitroanilid (suc-AAPF-pNA) bestimmt. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6 und einer Wellenlänge von 410 nm. Die Messzeit beträgt 5-10 min bei einem Messintervall von 20s bis 60s.

Die amylolytische Aktivität kann bestimmt werden wie folgt: Unter definierten Reaktionsbedingungen (Tris-maleatpuffer pH 6,5, 50°C, 15 min) werden die zu untersuchenden Proben mit 0,67% Stärke (FLUKA Nr. 85642; aus Kartoffel, löslich, vorbehandelt nach Zulkowsky (behandelt mit Glycerin bei 190°C)) als Substrat inkubiert. Durch Zugabe von Dinitrosalicylsäure und Erhitzen auf 100°C wird diese durch Glukose und andere reduzierende Zucker zu einem orangeroten Farbstoff reduziert, was nach Beendigung der Reaktion photometrisch bei einer Wellenlänge von 540nm bestimmt wird. Die der Färbung entsprechende Menge des freigesetzten Zuckers ist dabei ein Maß für die Enzymaktivität.

Die in Verfahrensschritt c) zu bestimmende relative Aktivität des in Verfahrensschritt b) veränderten hydrolytischen Enzyms ergibt sich dann gemäß [ermittelte Aktivität des in Verfahrensschritt b) veränderten hydrolytischen Enzyms in Anwesenheit der das hydrolytische Enzym stabilisierenden Komponente] geteilt durch [ermittelte Aktivität des in Verfahrensschritt b) veränderten hydrolytischen Enzyms in Abwesenheit der das hydrolytische Enzym stabilisierenden Komponente] multipliziert mit 100.

Die in Verfahrensschritt c) zu bestimmende relative Aktivität des Ausgangsenzyms ergibt sich dann gemäß [ermittelte Aktivität des Ausgangsenzyms in Anwesenheit der das hydrolytische Enzym stabilisierenden Komponente] geteilt durch [ermittelte Aktivität des Ausgangsenzyms in Abwesenheit der das hydrolytische Enzym stabilisierenden Komponente] multipliziert mit 100.

Die Bestimmung der Aktivitäten und demzufolge auch die Bestimmung der relativen Aktivitäten von hydrolytischem Enzym (stammend aus Verfahrensschritt b; Testansätze, jeweils mit und ohne stabilisierende Komponente) und Ausgangsenzym (Kontrollansätze, jeweils mit und ohne stabilisierende Komponente) erfolgt unter identischen Bedingungen, so dass die erhaltenen relativen Aktivitäten vergleichbar sind. Testansätze und Kontrollansätze unterscheiden sich folglich jeweils nur durch das eingesetzte Enzym, jeweils in An- bzw. Abwesenheit der stabilisierenden Komponente).

Für die Durchführung der Aktivitätsbestimmungen ist es ferner vorteilhaft, wenn die Konzentration der das hydrolytische Enzym stabilisierenden Komponente so gewählt wird, dass für das Ausgangsenzym im Kontrollansatz mit stabilisierender Komponente zwischen 90%-60% der Aktivität des Kontrollansatzes ohne stabilisierende Komponente vorliegt.

In Verfahrensschritt d) wird die in Verfahrensschritt c) bestimmte relative Aktivität des in Verfahrensschritt b) veränderten hydrolytischen Enzyms mit der relativen Aktivität des Ausgangsenzyms verglichen, und dann dasjenige hydrolytische Enzym ausgewählt, das eine verminderte relative Aktivität aufweist im Vergleich mit der relativen Aktivität des Ausgangsenzyms. Vorzugsweise wird dasjenige hydrolytische Enzym ausgewählt, das eine um mindestens 1%, 2%, 3%, 4%, 5%, 6%, 7%, 8%, 9%, 10%, 12,5%, 15%, 17,5%, 20%, 22,5%, 25%, 27,5%, 30%, 32,5%, 35%, 37,5% und 40% verminderte relative Aktivität im Vergleich mit der relativen Aktivität des Ausgangsenzyms aufweist.

Optional können die Verfahrensschritte b) bis d) wiederholt werden, bis das hydrolytische Enzym eine verminderte relative Aktivität aufweist im Vergleich mit der relativen Aktivität des Ausgangsenzyms. Vorzugsweise weist das

dann erhaltene hydrolytische Enzym eine um mindestens 1%, 2%, 3%, 4%, 5%, 6%, 7%, 8%, 9%, 10%, 12,5%, 15%, 17,5%, 20%, 22,5%, 25%, 27,5%, 30%, 32,5%, 35%, 37,5% und 40% verminderte relative Aktivität im Vergleich mit der relativen Aktivität des Ausgangsenzyms auf.

Ein derart erhaltenes hydrolytisches Enzym, insbesondere eine Protease, wird durch den reversiblen Inhibitor der das hydrolytische Enzym stabilisierenden Komponente verbessert inhibiert. Diese verbesserte inhibitorische Wirkung bewirkt in der Folge auch eine verbesserte Stabilisierung des hydrolytischen Enzyms. Eine verbesserte Hemmung des hydrolytischen Enzyms geht folglich mit dessen verbesserter Stabilisierung einher. Die verbesserte Stabilisierung kann in einem Lagertest wie vorstehend beschrieben bestimmt werden.

In einer weiteren Ausführungsform der Erfindung ist ein erfindungsgemäßes Verfahren dadurch gekennzeichnet, dass das hydrolytische Enzym eine Protease, Amylase, Cellulase, Glycosidase, Hemicellulase, Mannanase, Xylanase, Xyloglucanase, Xanthanase, Pektin-spaltendes Enzym, ß-Glucosidase, Carrageenase oder eine Lipase oder eine Mischung ist, die mindestens zwei dieser Enzyme umfasst.

Beispiele für Proteasen sind die Subtilisine BPN' aus Bacillus amyloliquefaciens und Carlsberg aus Bacillus licheniformis, die Protease PB92, die Subtilisine 147 und 309, die Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase® von dem Unternehmen Novozymes A/S, Bagsvæerd, Dänemark, erhältlich. Die Subtilisine 147 und 309 werden unter den Handelsnamen Esperase®, beziehungsweise Savinase® von dem Unternehmen Novozymes vertrieben. Von der Protease aus Bacillus lentus DSM 5483 leiten sich die unter der Bezeichnung BLAP® geführten Protease-Varianten ab. Weitere brauchbare Proteasen sind beispielsweise die unter den Handelsnamen Durazym®, Relase®, Everlase®, Nafizym®, Natalase®, Kannase® und Ovozyme® von dem Unternehmen Novozymes, die unter den Handelsnamen, Purafect®, Purafect® OxP, Purafect® Prime, Excellase® und Properase® von dem Unternehmen Danisco/Genencor, das unter dem Handelsnamen Protosol® von dem Unternehmen Advanced Biochemicals Ltd., Thane, Indien, das unter dem Handelsnamen Wuxi® von dem Unternehmen Wuxi Snyder Bioproducts Ltd., China, die unter den Handelsnamen Proleather® und Protease P® von dem Unternehmen Amano Pharmaceuticals Ltd., Nagoya, Japan, und das unter der Bezeichnung Proteinase K-16 von dem Unternehmen Kao Corp., Tokyo, Japan, erhältlichen Enzyme. Besonders bevorzugt eingesetzt werden auch die Proteasen aus Bacillus gibsonii und Bacillus pumilus, die offenbart sind in den internationalen Patentanmeldungen WO 08/086916 und WO 07/131656. Weitere vorteilhaft einsetzbare Proteasen sind offenbart in den Patentanmeldungen WO 91/02792, WO 08/007319, WO 93/18140, WO 01/44452, GB 1243784, WO 96/34946, WO 02/029024 und WO 03/057246. Weitere verwendbare Proteasen sind diejenigen, die in den Mikroorganismen Stenotrophomonas maltophilia, insbesondere Stenotrophomonas maltophilia K279a, Bacillus intermedius sowie Bacillus sphaericus natürlicherweise vorhanden sind.

Beispiele für Amylasen sind die $\alpha$-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl® und von dem Unternehmen Danisco/Genencor unter dem Namen Purastar®ST erhältlich. Weiterentwicklungsprodukte dieser $\alpha$-Amylase sind von dem Unternehmen Novozymes unter den Handelsnamen Duramyl® und Termamyl®ultra, von dem Unternehmen Danisco/Genencor unter dem Namen Purastar®OxAm und von dem Unternehmen Daiwa Seiko Inc., Tokyo, Japan, als Keistase® erhältlich. Die $\alpha$-Amylase von Bacillus amyloliquefaciens wird von dem Unternehmen Novozymes unter dem Namen BAN® vertrieben, und abgeleitete Varianten von der $\alpha$-Amylase aus Bacillus stearothermophilus unter den Namen BSG® und Novamyl®, ebenfalls von dem Unternehmen Novozymes. Des Weiteren sind für diesen Zweck die $\alpha$-Amylase aus Bacillus sp. A 7-7 (DSM 12368) und die Cyclodextrin-Glucanotransferase (CGTase) aus Bacillus agaradherens (DSM 9948) hervorzuheben. Ferner sind die amylolytischen Enzyme einsetzbar, die in den internationalen Patentanmeldungen WO 03/002711, WO 03/054177 und WO07/079938 offenbart sind. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der $\alpha$-Amylase aus Aspergillus niger und A. oryzae geeignet. Weitere vorteilhaft einsetzbare Handelsprodukte sind beispielsweise die Amylase-LT® und Stainzyme® oder Stainzyme ultra® bzw. Stainzyme plus®, letztere ebenfalls von dem Unternehmen Novozymes. Auch durch Punktmutationen erhältliche Varianten dieser Enzyme können erfindungsgemäß eingesetzt werden.

Beispiele für Cellulasen (Endoglucanasen, EG) ist die pilzliche, Endoglucanase(EG)-reiche Cellulase-Präparation beziehungsweise deren Weiterentwicklungen, die von dem Unternehmen Novozymes unter dem Handelsnamen Celluzyme® angeboten wird. Die ebenfalls von dem Unternehmen Novozymes erhältlichen Produkte Endolase® und Carezyme® basieren auf der 50 kD-EG, beziehungsweise der 43 kD-EG aus Humicola insolens DSM 1800. Weitere einsetzbare Handelsprodukte dieses Unternehmens sind Cellusoft®, Renozyme® und Celluclean®. Weiterhin einsetzbar sind beispielsweise Cellulasen, die von dem Unternehmen AB Enzymes, Finnland, unter den Handelsnamen Ecostone® und Biotouch® erhältlich sind, und die zumindest zum Teil auf der 20 kD-EG aus Melanocarpus basieren. Weitere Cellulasen von dem Unternehmen AB Enzymes sind Econase® und Ecopulp®. Weitere geeignete Cellulasen sind aus

Bacillus sp. CBS 670.93 und CBS 669.93, wobei die aus Bacillus sp. CBS 670.93 von dem Unternehmen Danisco/Genencor unter dem Handelsnamen Puradax® erhältlich ist. Weitere verwendbare Handelsprodukte des Unternehmens Danisco/Genencor sind "Genencor detergent cellulase L" und IndiAge®Neutra.

Weitere bevorzugte hydrolytische Enzyme sind solche, die unter dem Begriff Glycosidasen (E.C. 3.2.1.X) zusammengefasst sind. Hierzu zählen insbesondere Arabinasen, Fucosidasen, Galactosidasen, Galactanasen, Arabico-Galactan-Galactosidasen, Mannanasen (auch bezeichnet als Mannosidasen oder Mannasen), Glucuronosidasen, Agarase, Carrageenasen, Pullulanasen, ß-Glucosidasen, Xyloglucanasen (Xylanasen), Xanthanasen und Pektin-abbauende Enzyme (Pektinasen). Bevorzugte Gylcosidasen werden auch unter dem Begriff Hemicellulasen zusammengefasst. Zu Hemicellulasen zählen insbesondere Mannanasen, Xyloglucanasen (Xylanasen), ß-Glucosidasen und Carrageenasen sowie ferner Pektin-spaltende Enzyme, Pullulanasen und ß-Glucanasen.

Pektin-spaltende Enzyme (Pektinasen) im Sinne der Erfindung sind Enzyme, die Pektine und/oder andere Galakturonane spalten. Bei Pektinen handelt es sich um Polysaccharide, deren Hauptbestandteil $\alpha$-D-Galacturonsäure als Monomer ist, vorzugsweise zu mindestens 50 Gew.-% und besonders bevorzugt zu mindestens 65 Gew.-%. Diese Galacturonsäure-Monomere sind über $\alpha$-1,4-, manchmal auch zu einem geringen Anteil über $\beta$-1,4-giycosidische Bindungen, miteinander verbunden und bilden das Rückgrat des Pektinmoleküls, das periodisch durch 1,2-Bindungen mit $\alpha$-L-Rhamnose unterbrochen wird. Ein Pektin ist folglich eine Rhamno-galacturonsäure. Eine Pektin-spaltendes Enzym ist folglich insbesondere ein Enzym, das die Hydrolyse von 1,4-$\alpha$-D-galaktosiduronischen Bindungen katalysiert.

Innerhalb der EC-Klassifikation der Enzyme, dem numerischen Klassifikationssystem für Enzyme, sind die Pektin-spaltenden Enzyme insbesondere zugehörig zu den Enzymklassen (engl. "Enzyme Commission number") EC 3.1.1.11, EC 3.2.1.15, EC 3.2.1.67 und EC 3.2.1.82 und zählen folglich zur dritten der sechs Enzymhauptklassen, den Hydrolasen (E.C.3.-.-.-), hierunter zu den Glycosylasen (E.C. 3.2.-.-) und wiederum hierunter zu den Glycosidasen (E.C. 3.2.1.-), d.h. Enzymen, die O- und/oder S-Glycosyl-Verbindungen hydrolysieren. Pektin-spaltende Enyzme wirken folglich insbesondere gegen Rückstände auf Geschirr, die Pektinsäure und/oder andere Galakturonane enthalten, und katalysieren deren Hydrolyse.

Zu den Pektin-spaltenden Enzymen werden im Rahmen der vorliegenden Erfindung ebenfalls Enzyme gezählt mit den Bezeichnungen Pektinase, Pektatlyase, Pektinesterase, Pektindemethoxylase, Pektinmethoxylase, Pektinmethylesterase, Pektase, Pektinmethylesterase, Pektinoesterase, Pektinpektylhydrolase, Pektindepolymerase, Endopolygalacturonase, Pektolase, Pektinhydrolase, Pektin-Polygalacturonase, Endo-Polygalacturonase, Poly-$\alpha$-1,4-Galacturonid Glycanohydrolase, Endogalacturonase, Endo-D-galacturonase, Galacturan 1,4-$\alpha$-Galacturonidase, Exopolygalacturonase, Poly(galacturonat) Hydrolase, Exo-D-Galacturonase, Exo-D-Galacturonanase, Exopoly-D-Galacturonase, Exo-poly-$\alpha$-Galacturonosidase, Exopolygalacturonosidase oder Exopolygalacturanosidase.

Beispiele für diesbezüglich geeignete Enzyme sind beispielsweise unter den Namen Gamanase®, Pektinex AR®, X-Pect® oder Pectaway® von dem Unternehmen Novozymes, unter dem Namen Rohapect UF®, Rohapect TPL®, Rohapect PTE100®, Rohapect MPE®, Rohapect MA plus HC, Rohapect DA12L®, Rohapect 10L®, Rohapect B1L® von dem Unternehmen AB Enzymes und unter dem Namen Pyrolase® von dem Unternehmen Diversa Corp., San Diego, CA, USA erhältlich.

Ein weiteres Beispiel ist die aus Bacillus subtilis gewonnene ß-Glucanase, die unter dem Namen Cereflo® von dem Unternehmen Novozymes erhältlich ist. Erfindungsgemäß besonders bevorzugte Glycosidasen und hierunter Hemicellulasen sind Mannanasen, welche beispielsweise unter den Handelsnamen Mannaway® von dem Unternehmen Novozymes oder Purabrite® von dem Unternehmen Danisco/Genencor vertrieben werden.

Beispiele für Lipasen oder Cutinasen sind die ursprünglich aus Humicola lanuginosa (Thermomyces lanuginosus) erhältlichen beziehungsweise daraus weiterentwickelten Lipasen, insbesondere solche mit dem Aminosäureaustausch D96L. Sie werden beispielsweise von dem Unternehmen Novozymes unter den Handelsnamen Lipolase®, Lipolase®Ultra, LipoPrime®, Lipozyme® und Lipex® vertrieben. Eine weitere vorteilhaft einsetzbare Lipase ist unter dem Handelsnamen Lipoclean® von dem Unternehmen Novozymes erhältlich. Des Weiteren sind beispielsweise die Cutinasen einsetzbar, die ursprünglich aus Fusarium solani pisi und Humicola insolens isoliert worden sind. Ebenso brauchbare Lipasen sind von dem Unternehmen Amano unter den Bezeichnungen Lipase CE®, Lipase P®, Lipase B®, beziehungsweise Lipase CES®, Lipase AKG®, Bacillis sp. Lipase®, Lipase AP®, Lipase M-AP® und Lipase AML® erhältlich. Von dem Unternehmen Danisco/Genencor sind beispielsweise die Lipasen beziehungsweise Cutinasen einsetzbar, deren Ausgangsenzyme ursprünglich aus Pseudomonas mendocina und Fusarium solanii isoliert worden sind. Als weitere wichtige Handelsprodukte sind die ursprünglich von dem Unternehmen Gist-Brocades (inzwischen Danisco/Genencor) vertriebenen Präparationen M1 Lipase® und Lipomax® und die von dem Unternehmen Meito Sangyo KK, Japan, unter den Namen Lipase MY-30®, Lipase OF® und Lipase PL® vertriebenen Enzyme zu erwähnen, ferner das Produkt Lumafast® von dem Unternehmen Danisco/Genencor.

Die im Rahmen der vorliegenden Erfindung einzusetzenden Enzyme können beispielsweise ursprünglich aus Mikroorganismen, etwa der Gattungen Bacillus, Streptomyces, Humicola, oder Pseudomonas, stammen und/oder nach an sich bekannten biotechnologischen Verfahren durch geeignete Mikroorganismen produziert werden, etwa durch transgene Expressionswirte, beispielsweise der Gattungen Escherichia, Bacillus, oder durch filamentöse Pilze.

Besonders bevorzugt ist das hydrolytische Enzym eine Protease und/oder eine Amylase. Ganz besonders bevorzugt ist das hydrolytische Enzym eine Protease. Unter den Proteasen bevorzugt ist eine Serinprotease, weiter bevorzugt eine Subtilase und ganz besonders bevorzugt ein Subtilisin. Es hat sich gezeigt, dass Proteasen, insbesondere solche Proteasen, durch ein erfindungsgemäßes Verfahren besonders gut an eine das hydrolytische Enzym stabilisierende Komponente angepasst werden können, insbesondere in einer flüssigen Tensidzubereitung. Entsprechendes gilt für Amylasen, die ein erfindungsgemäßes Verfahren besonders gut an eine das hydrolytische Enzym stabilisierende Komponente angepasst werden können, insbesondere in einer flüssigen Tensidzubereitung. Denn insbesondere für Wasch-, Reinigungs- oder Desinfektionsmittel ist die Lagerstabilität der Enzyme und insbesondere auch die von Proteasen und/oder Amylasen ein generelles Problem.

Im Hinblick auf Proteasen als bevorzugte hydrolytische Enzyme umfasst die stabilisierende Komponente folglich einen reversiblen Proteaseinhibitor. Proteasen sind häufig für die mangelnde Enzymstabilität in flüssigen Zubereitungen verantwortlich, indem sie die enthaltenen Enzyme hydrolysieren. Mit einem reversiblen Proteaseinhibitor lässt sich folglich die Enzymstabilität in flüssigen Zubereitungen verbessern, indem die proteolytische Aktivität in der Zubereitung gehemmt wird und die Hemmung erst wieder bei Bedarf der proteolytischen Aktivität aufgehoben wird, beispielsweise durch Verdünnen der Zubereitung, insbesondere mit Wasser. Es muss sich um reversible Proteaseinhibitoren handeln, da die Proteaseaktivität nur vorübergehend, insbesondere während der Lagerung, nicht aber mehr während der vorgesehenen Anwendung der flüssigen Zubereitung, beispielsweise während des Reinigungsprozesses, unterbunden werden soll. Eine stärkere Inhibierung der Proteasen in der flüssigen Zubereitung führt zu einer stärkeren Stabilisierung der Proteasen sowie von weiteren Enzymen. Erfindungsgemäße Proteaseinhibitoren hemmen folglich reversibel die Proteaseaktivität und bewirken auf diese Weise eine Stabilisierung der Proteasen sowie von weiteren Enzymen, insbesondere auf Grund der durch den Inhibitor verminderten proteolytischen Aktivität der Proteasen. Eine stärkere Inhibierung der Proteasen führt zu einer stärkeren Stabilisierung der Proteasen sowie von weiteren Enzymen, beispielsweise auf Grund des gesteigerten Schutzes vor proteolytischem Abbau. In einer weiteren bevorzugten Ausführungsform der Erfindung ist ein erfindungsgemäßes Verfahren dadurch gekennzeichnet, dass das hydrolytische Enzym eine Protease ist und die das hydrolytische Enyzm stabilisierende Komponente einen reversiblen Proteaseinhibitor umfasst.

In einer weiteren Ausführungsform der Erfindung ist ein erfindungsgemäßes Verfahren dadurch gekennzeichnet, dass die Protease an einer Position verändert wird, die der Position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274 oder 275 in SEQ ID NO. 1, zugeordnet in einem Alignment mit SEQ ID NO. 1, entspricht, oder dass die Amylase an einer Position verändert wird, die der Position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484 oder 485 in SEQ ID NO. 2, zugeordnet in einem

Alignment mit SEQ ID NO. 2, entspricht.

Die Zuordnung der Aminosäurepositionen wie auch die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F.,Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Softwarepaket Vector NTI® Advance 10.3.0 (Invitrogen Corporation, 1600 Faraday Avenue, Carlsbad, Kalifornien, USA) mit den vorgegebenen Standard (Default)-Parametern erstellt.

Solch ein Vergleich erlaubt einerseits eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw. Funktionen ausüben. Daher kann die Ähnlichkeit der verglichenen Sequenzen auch Prozent Homologie oder Prozent Ähnlichkeit angegeben sein. Soweit nicht anders angegeben beziehen sich Identitäts- bzw. Homologieangaben in der vorliegenden Anmeldung auf die Gesamtlänge der jeweils angegebenen Nukleinsäure- oder Aminosäuresäuresequenz.

Andererseits erlaubt ein Sequenzvergleich (Alignment) die genaue Bestimmung der Position in einer Aminosäuresequenz, die einer bestimmten Position in einer anderen Aminosäuresequenz zugeordnet ist. Folglich kann mit Hilfe eines Alignments genau ermittelt werden, welche Aminosäure in welcher Position in der erfindungsgemäß zu verändernden Protease einer der vorstehend genannten Positionen in den genannten Referenzsequenzen entspricht. SEQ ID NO. 1 ist die Aminosäuresequenz der Protease BPN', SEQ ID NO. 2 ist die Aminosäuresequenz der Amylase, die offenbart ist in SEQ ID NO. 4 der WO 2000/60060.

In einer weiteren bevorzugten Ausführungsform der Erfindung ist das erfindungsgemäße Verfahren dadurch gekennzeichnet, dass die das hydrolytische Enyzm stabilisierende Komponente in einer Menge von 0,000001 bis 10 Gew.-%, von 0,0001 bis 5 Gew.-%, von 0,001 bis 1 Gew.-% und besonders bevorzugt von 0,002 bis 0,35 Gew.-% in der flüssigen Zubereitung enthalten ist, und/oder das hydrolytische Enzym in einer Menge von 1 x $10^{-8}$-10 Gew.-%, von 0,00001-2 Gew.-%, von 0,001-1 Gew.-%, von 0,007 bis 0,8 Gew.-%, von 0,025 bis 0,5 Gew.-% und besonders bevorzugt von 0,04 bis 0,38 Gew.-%, bezogen auf den Gesamtproteingehalt des hydrolytischen Enzyms. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden.

In einer weiteren bevorzugten Ausführungsform der Erfindung ist ein erfindungsgemäßes Verfahren dadurch gekennzeichnet, dass die flüssige Zubereitung in Verfahrensschritt c) eine Tensidzubereitung ist.

Unter einer Tensidzubereitung ist im Rahmen der vorliegenden Erfindung jegliche Art von Zusammensetzung zu verstehen, die mindestens ein Tensid enthält. Vorzugsweise enthält eine derartige Zusammensetzung ein Tensid wie weiter unten beschrieben.

Als flüssige Tensidzubereitungen können hierbei alle flüssigen bzw. fließfähigen Darreichungsformen dienen. "Fließfähig" im Sinne der vorliegenden Anmeldung sind dabei Zubereitungen, welche gießbar sind und Viskositäten bis hin zu mehreren 10.000 mPas aufweisen können. Die Viskosität kann mit üblichen Standardmethoden (beispielsweise Brookfield-Viskosimeter LVT-II bei 20 U/min und 20°C, Spindel 3) gemessen werden und liegt vorzugsweise im Bereich von 5 bis 10000 mPas. Bevorzugte Mittel haben Viskositäten von 10 bis 8000 mPas, wobei Werte zwischen 120 bis 3000 mPas besonders bevorzugt sind. Eine flüssige Tensidzubereitung im Rahmen der vorliegenden Erfindung kann daher auch gelförmig oder pastenförmig sein, sie kann als homogene Lösung oder Suspension vorliegen, sowie beispielsweise versprühbar oder in sonstigen üblichen Darreichungsformen konfektioniert sein.

Eine erfindungsgemäße flüssige Tensidzubereitung kann als solche oder nach Verdünnen mit Wasser eingesetzt werden, insbesondere zur Reinigung von Textilien und/oder harten Oberflächen. Eine solche Verdünnung kann leicht hergestellt werden, indem eine abgemessene Menge der Tensidzubereitung in einer weiteren Menge Wasser

verdünnt wird in bestimmten Gewichtsverhältnissen von Tensidzubereitung : Wasser und optional diese Verdünnung geschüttelt wird, um eine gleichmäßige Verteilung der Tensidzubereitung im Wasser sicherzustellen. Mögliche Gewichts- oder Volumenverhältnisse der Verdünnungen sind von 1:0 Tensidzubereitung : Wasser bis 1:10000 oder 1:20000 Tensidzubereitung : Wasser, vorzugsweise von 1:10 bis 1:2000 Tensidzubereitung : Wasser.

Eine Tensidzubereitung im Sinne der vorliegenden Erfindung kann daher auch die Wasch- bzw. Reinigungsflotte selbst sein. Unter Wasch- bzw. Reinigungsflotte wird diejenige das Wasch- oder Reinigungsmittel enthaltende Gebrauchslösung verstanden, die auf Textilien oder Gewebe (Waschflotte) bzw. harte Oberflächen (Reinigungsflotte) einwirkt und damit mit den auf Textilien bzw. Geweben oder harten Oberflächen vorhandenen Anschmutzungen in Kontakt kommt. Üblicherweise entsteht die Wasch- bzw. Reinigungsflotte, wenn der Wasch- oder Reinigungsvorgang beginnt und das Wasch- oder Reinigungsmittel beispielsweise in einer Waschmaschine oder in einem anderen geeigneten Behältnis mit Wasser verdünnt wird.

In einer bevorzugten Ausführungsform ist die Tensidzubereitung ein Wasch-, Reinigungs- oder Desinfektionsmittel. Zu den Waschmitteln zählen alle denkbaren Waschmittelarten, insbesondere Waschmittel für Textilien, Teppiche oder Naturfasern. Sie können für die manuelle und/oder auch die maschinelle Anwendung vorgesehen sein. Zu den Waschmitteln zählen ferner Waschhilfsmittel, die bei der manuellen oder maschinellen Textilwäsche zum eigentlichen Waschmittel hinzudosiert werden, um eine weitere Wirkung zu erzielen. Zu den Reinigungsmitteln werden alle, ebenfalls in sämtlichen genannten Darreichungsformen vorkommenden Mittel zur Reinigung und/oder Desinfektion harter Oberflächen, manuelle und maschinelle Geschirrspülmittel, Teppichreiniger, Scheuermittel, Glasreiniger, WC-Duftspüler, usw. gezählt. Textilvor- und Nachbehandlungsmittel sind schließlich auf der einen Seite solche Mittel, mit denen das Wäschestück vor der eigentlichen Wäsche in Kontakt gebracht wird, beispielsweise zum Anlösen hartnäckiger Verschmutzungen, auf der anderen Seite solche, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet. Desinfektionsmittel sind beispielsweise Händedesinfektionsmittel, Flächendesinfektionsmittel und Instrumentendesinfektionsmittel, die ebenfalls in den genannten Darreichungsformen vorkommen können. Ein Desinfektionsmittel bewirkt vorzugsweise eine Keimreduktion um einen Faktor von mindestens $10^4$, das heißt dass von ursprünglich 10.000 vermehrungsfähigen Keimen (so genannte koloniebildende Einheiten - KBE) nicht mehr als ein Einziger überlebt, wobei Viren diesbezüglich nicht als Keime gelten, da sie kein Zytoplasma und keinen eigenen Stoffwechsel aufweisen. Bevorzugte Desinfektionsmittel bewirken eine Keimreduktion um einen Faktor von mindestens $10^5$.

Als Tensid(e) können anionische, nichtionische, zwitterionische und/oder amphotere Tenside eingesetzt werden. Bevorzugt sind aus anwendungstechnischer Sicht Mischungen aus anionischen und nichtionischen Tensiden. Der Gesamttensidgehalt der flüssigen Tensidzubereitung liegt vorzugsweise unterhalb von 60 Gew.-% und besonders bevorzugt unterhalb von 45 Gew.-%, bezogen auf die gesamte flüssige Tensidzubereitung.

Geeignete nichtionische Tenside umfassen alkoxylierte Fettalkohole, alkoxylierte Fettsäurealkylester, Fettsäureamide, alkoxylierte Fettsäureamide, Polyhydroxyfettsäureamide, Alkylphenolpolyglycolether, Aminoxide, Alkylpolyglucoside und Mischungen daraus.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise $C_{12-14}$-Alkohole mit 3 EO, 4 EO oder 7 EO, $C_{9-11}$-Alkohol mit 7 EO, $C_{13-15}$-Alkohole mit 3 EO, 5 EO, 7 EO oder 8 EO, $C_{12-18}$-Alkohole mit 3 EO, 5 EO oder 7 EO und Mischungen aus diesen, wie Mischungen aus $C_{12-14}$-Alkohol mit 3 EO und $C_{12-18}$-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem $C_{16-18}$-Fettalkohol mit 7 EO und 2-Propylheptanol mit 7 EO. Insbesondere bevorzugt enthält die Tensidzubereitung einen $C_{12-18}$-Fettalkohol mit 7 EO oder einen $C_{13-15}$-Oxoalkohol mit 7 EO als nichtionisches Tensid.

Der Gehalt an nichtionischen Tensiden beträgt bevorzugt 3 bis 40 Gew.-%, vorzugsweise 5 bis 30 Gew.-% und insbesondere 7 bis 20 Gew.-%, jeweils bezogen auf die gesamte Tensidzubereitung.

Neben den nichtionischen Tensiden kann die Tensidzubereitung auch anionische Tenside enthalten. Als anionisches Tensid werden vorzugsweise Sulfonate, Sulfate, Seifen, Alkylphosphate, anionische Silikontenside und Mischungen daraus eingesetzt.

Als Tenside vom Sulfonat-Typ kommen dabei vorzugsweise $C_{9-13}$-Alkylbenzolsulfonate, Olefinsulfonate, d.h. Gemische aus Alken- und Hydroxyalkansulfonaten sowie Disulfonaten, wie man sie beispielsweise aus $C_{12-18}$-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch $C_{12-18}$-Alkansulfonate und die Ester von $\alpha$-Sulfofettsäuren (Estersulfonate), zum Beispiel die $\alpha$-sulfonierten Methylester der hydrierten Kokos-, Palmkern- oder Talgfettsäuren.

Als Alk(en)ylsulfate werden die Alkali- und insbesondere die Natriumsalze der Schwefelsäurehalbester der $C_{12}$-$C_{18}$-Fettalkohole, beispielsweise aus Kokosfettalkohol, Talgfettalkohol, Lauryl-, Myristyl-, Cetyl- oder Stearylalkohol oder der $C_{10}$-$C_{20}$-Oxoalkohole und diejenigen Halbester sekundärer Alkohole dieser Kettenlängen bevorzugt. Aus waschtechnischem Interesse sind die $C_{12}$-$C_{16}$-Alkylsulfate und $C_{12}$-$C_{15}$-Alkylsulfate sowie $C_{14}$-$C_{15}$-Alkylsulfate bevorzugt. Auch 2,3-Alkylsulfate sind geeignete anionische Tenside.

Auch die Schwefelsäuremonoester der mit 1 bis 6 Mol Ethylenoxid ethoxylierten geradkettigen oder verzweigten $C_{7-21}$-Alkohole, wie 2-Methyl-verzweigte $C_{9-11}$-Alkohole mit im Durchschnitt 3,5 Mol Ethylenoxid (EO) oder $C_{12-18}$-Fettalkohole mit 1 bis 4 EO, sind geeignet.

Auch bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Magnesium- oder Ammoniumsalze vorliegen. Vorzugsweise liegen die anionischen Tenside in Form ihrer Natriumsalze vor. Weitere bevorzugte Gegenionen für die anionischen Tenside sind auch die protonierten Formen von Cholin, Triethylamin oder Methylethylamin.

Der Gehalt einer Tensidzubereitung an anionischen Tensiden kann 1 bis 40 Gew.-%, vorzugsweise 5 bis 30 Gew.-% und ganz besonders bevorzugt 10 bis 25 Gew.-%, jeweils bezogen auf die gesamte Tensidzubereitung, betragen.

In einer weiteren Ausführungsform ist die Tensidzubereitung dadurch gekennzeichnet, dass sie ferner mindestens einen weiteren Inhaltsstoff umfasst, der ausgewählt ist aus der Gruppe bestehend aus Gerüststoff (Builder), nichtwässriges Lösungsmittel, Säure, wasserlösliches Salz, Verdickungsmittel, desinfizierender Inhaltsstoff sowie Kombinationen hiervon.

Das Hinzufügen von einem oder mehreren der weiteren Inhaltsstoff(e) erweist sich als vorteilhaft, da hierdurch eine weiter verbesserte Reinigungsleistung und/oder Desinfektion erreicht wird. Vorzugsweise beruht die verbesserte Reinigungsleistung und/oder Desinfektion auf einem synergistischen Zusammenwirken von mindestens zwei Inhaltsstoffen. Insbesondere durch die Kombination des hydrolytischen Enzyms, vorzugsweise einer Protease, mit einem der nachstehend beschriebenen Gerüststoffe (Builder) und/oder mit einem der nachfolgend beschriebenen nichtwässrigen Lösungsmittel und/oder mit einer der nachfolgend beschriebenen Säuren und/oder mit einem der nachstehend beschriebenen wasserlöslichen Salze und/oder mit einem der nachfolgend beschriebenen Verdickungsmittel und/oder mit einem der nachfolgend beschriebenen desinfizierenden Inhaltsstoffen kann eine solche Synergie erreicht werden.

Als Gerüststoffe (Builder), die in der Tensidzubereitung enthalten sein können, sind insbesondere Silikate, Aluminiumsilikate (insbesondere Zeolithe), Carbonate, Salze organischer Di- und Polycarbonsäuren sowie Mischungen dieser Stoffe zu nennen.

Organische Gerüststoffe, welche in der Tensidzubereitung vorhanden sein können, sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), Methylglycindiessigsäure (MGDA) und deren Abkömmlinge sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g / mol.

Geeignete Polymere sind insbesondere Polyacrylate, die bevorzugt eine Molekülmasse von 1.000 bis 15.000 g / mol aufweisen. Aufgrund ihrer überlegenen Löslichkeit können aus dieser Gruppe wiederum die kurzkettigen Polyacrylate, die Molmassen von 1.000 bis 10.000 g / mol, und besonders bevorzugt von 1.000 bis 5.000 g / mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Bevorzugt werden allerdings lösliche Gerüststoffe, wie beispielsweise Citronensäure, oder Acrylpolymere mit

einer Molmassen von 1.000 bis 5.000 g / mol bevorzugt in der flüssigen Tensidzubereitung eingesetzt.

Bei den für polymere Polycarboxylate angegebenen Molmassen handelt es sich im Sinne dieser Schrift um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, Tensidzubereitungen eingesetzt.

Die erfindungsgemäßen Tensidzubereitungen sind flüssig und enthalten vorzugsweise Wasser als Hauptlösungsmittel. Daneben oder alternativ können der Tensidzubereitung nichtwässrige Lösungsmittel zugesetzt werden. Geeignete nichtwässrige Lösungsmittel umfassen ein- oder mehrwertige Alkohole, Alkanolamine oder Glykolether, sofern sie im angegebenen Konzentrationsbereich mit Wasser mischbar sind. Vorzugsweise werden die Lösungsmittel ausgewählt aus Ethanol, n-Propanol, i-Propanol, Butanolen, Glykol, Propandiol, Butandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Diisopropylenglykolmonomethylether, Di-isopropylenglykolmonoethylether, Methoxytriglykol, Ethoxytriglykol, Butoxytriglykol, 1-Butoxyethoxy-2-propanol, 3-Methyl-3-methoxybutanol, Propylenglykol-t-butylether, Di-n-octylether sowie Mischungen dieser Lösungsmittel. Es ist allerdings bevorzugt, dass die Tensidzubereitung ein Polyol als nicht-wässriges Lösungsmittel enthält. Das Polyol kann insbesondere Glycerin, 1,2-Propandiol, 1,3-Propandiol, Ethylenglycol, Diethylenglycol und/oder Dipropylenglycol umfassen. Insbesondere bevorzugt enthält die Tensidzubereitung eine Mischung aus einem Polyol und einem einwertigen Alkohol. Nichtwässrige Lösungsmittel können in der Tensidzubereitung in Mengen zwischen 0,5 und 15 Gew.-%, bevorzugt aber unter 12 Gew.-% und eingesetzt werden.

Zur Einstellung eines gewünschten, sich durch die Mischung der übrigen Komponenten nicht von selbst ergebenden pH-Werts können die Tensidzubereitungen system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den Tensidzubereitungen in Mengen von vorzugsweise nicht über 20 Gew.-%, insbesondere von 1,2 Gew.-% bis 17 Gew.-%, enthalten.

Eine Tensidzubereitung im Sinne der Erfindung kann weiterhin ein oder mehrere wasserlösliche Salze enthalten, die beispielsweise zur Viskositätseinstellung dienen. Es kann sich dabei um anorganische und/oder organische Salze handeln. Einsetzbare anorganische Salze sind dabei vorzugsweise ausgewählt aus der Gruppe umfassend farblose wasserlösliche Halogenide, Sulfate, Sulfite, Carbonate, Hydrogencarbonate, Nitrate, Nitrite, Phosphate und/oder Oxide der Alkalimetalle, der Erdalkalimetalle, des Aluminiums und/oder der Übergangsmetalle; weiterhin sind Ammoniumsalze einsetzbar. Besonders bevorzugt sind dabei Halogenide und Sulfate der Alkalimetalle; vorzugsweise ist das anorganische Salz daher ausgewählt aus der Gruppe umfassend Natriumchlorid, Kaliumchlorid, Natriumsulfat, Kaliumsulfat sowie Gemische derselben. Einsetzbare organische Salze sind beispielsweise farblose wasserlösliche Alkalimetall-, Erdalkalimetall-, Ammonium-, Aluminium- und/oder Übergangsmetallsalze der Carbonsäuren. Vorzugsweise sind die Salze ausgewählt aus der Gruppe umfassend Formiat, Acetat, Propionat, Citrat, Malat, Tartrat, Succinat, Malonat, Oxalat, Lactat sowie Gemische derselben.

Zur Verdickung kann eine erfindungsgemäße Tensidzubereitung ein oder mehrere Verdickungsmittel enthalten. Bevorzugt ist das Verdickungsmittel ausgewählt aus der Gruppe umfassend Xanthan, Guar, Carrageenan, Agar-Agar, Gellan, Pektin, Johannisbrotkernmehl und Mischungen daraus. Diese Verbindungen sind auch in Gegenwart von anorganischen Salzen effektive Verdickungsmittel. In einer besonders bevorzugten Ausführungsform enthält die Tensidzubereitung Xanthan als Verdickungsmittel, da Xanthan auch in Gegenwart von hohen Salzkonzentrationen effektiv verdickt und eine makroskopische Auftrennung der kontinuierlichen Phase verhindert. Zusätzlich stabilisiert das Verdickungsmittel die kontinuierliche, tensidarme Phase und verhindert eine makroskopische Phasenseparation.

Alternativ oder ergänzend können auch (Meth)Acrylsäure(co)polymere als Verdickungsmittel eingesetzt werden. Geeignete Acryl- und Methacryl(co)polymere umfassen beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung gemäß "International Dictionary of Cosmetic Ingredients" der "The Cosmetic, Toiletry and Fragrance Association (CTFA)": Carbomer), die auch als Carboxyvinylpolymere bezeichnet werden. Solche Polyacrylsäuren sind unter anderem unter den Handelsnamen Polygel® und Carbopol® erhältlich. Weiterhin sind beispielsweise folgende Acrylsäure-Copolymere geeignet: (i) Copolymere von zwei oder mehr Monomeren aus der Gruppe der Acrylsäure, Methacrylsäure und ihrer einfachen, vorzugsweise mit $C_{1-4}$-Alkanolen gebildeten, Ester (INCI Acrylates

Copolymer), die beispielsweise unter den Handelsnamen Aculyn®, Acusol® oder Tego® Polymer erhältlich sind; (ii) vernetzte hochmolekulare Acrylsäure-Copolymere, zu denen etwa die mit einem Allylether der Saccharose oder des Pentaerythrits vernetzten Copolymere von $C_{10-30}$-Alkylacrylaten mit einem oder mehreren Monomeren aus der Gruppe der Acrylsäure, Methacrylsäure und ihrer einfachen, vorzugsweise mit $C_{1-4}$-Alkanolen gebildeten, Ester (INCI Acrylates/$C_{10-30}$ Alkyl Acrylate Crosspolymer) gehören und die beispielsweise unter dem Handelsnamen Carbopol® erhältlich sind. Weitere geeignete Polymere sind (Meth)Acrylsäure(co)polymere des Typs Sokalan®.

Es kann bevorzugt sein, dass die erfindungsgemäße Tensidzubereitung ein (Meth)Acrylsäure(co)polymer in Kombination mit einem weiteren Verdickungsmittel, vorzugsweise Xanthan, enthält. Die Tensidzubereitung kann 0,05 bis 1,5 Gew.-% und vorzugsweise 0,1 bis 1 Gew.-%, jeweils bezogen auf die gesamte Tensidzubereitung, Verdickungsmittel enthalten. Die Menge an eingesetztem Verdickungsmittel ist dabei abhängig von der Art des Verdickungsmittels und dem gewünschten Grad der Verdickung.

Unter einem desinfizierenden Inhaltsstoff werden insbesondere Inhaltsstoffe verstanden, die eine antimikrobielle oder antivirale Wirksamkeit besitzen, also Keime abtöten. Die keimabtötende Wirkung ist dabei abhängig von dem Gehalt des desinfizierenden Inhaltsstoffes in der Tensidzubereitung, wobei die keimabtötende Wirkung mit abnehmendem Gehalt an desinfizierendem Inhaltsstoff bzw. zunehmender Verdünnung der Tensidzubereitung abnimmt.

Ein bevorzugter desinfizierender Inhaltsstoff ist Ethanol oder Propanol. Diese einwertigen Alkohole werden aufgrund ihrer Lösemitteleigenschaften und ihrer keimtötenden Wirkung häufig in Desinfektionsmitteln und auch in Reinigungsmitteln allgemein eingesetzt. Dabei umfasst der Begriff "Propanol" sowohl das 1-Propanol (n-Propanol) als auch das 2-Propanol ("Isopropanol"). Ethanol und/oder Propanol ist beispielsweise in einer Menge von insgesamt 10 bis 65 Gew.-%, vorzugsweise 25 bis 55 Gew.-% in der Tensidzubereitung enthalten. Ein weiterer bevorzugter desinfizierender Inhaltsstoff ist Teebaumöl. Hierbei handelt es sich um das ätherische Öl des Australischen Teebaums (Melaleuca alternifolia), einem in New South Wales und Queensland beheimateten immergrünen Strauch aus der Gattung Myrtenheiden (Melaleuca), sowie weiterer Teebaum-Arten aus verschiedenen Gattungen (z.B. Baeckea, Kunzea und Leptospermum) in der Familie der Myrtengewächse (Myrtaceae). Das Teebaumöl wird durch Wasserdampfdestillation aus den Blättern und Zweigspitzen dieser Bäume gewonnen und ist ein Gemisch aus ca. 100 Substanzen; zu den Hauptbestandteilen zählen (+)-Terpinen-4-ol, $\alpha$-Terpinen, Terpinolen, Terpineol, Pinen, Myrcen, Phellandren, p-Cymen, Limonen und 1,8-Cineol. Teebaumöl ist beispielsweise in einer Menge von 0,05 bis 10 Gew.-%, vorzugsweise 0,1 bis 5,0 Gew.-%, in der viruziden Behandlungslösung enthalten. Ein weiterer bevorzugter desinfizierender Inhaltsstoff ist Milchsäure. Die Milchsäure oder 2-Hydroxypropionsäure ist ein Gärungsprodukt, das von verschiedenen Mikroorganismen erzeugt wird. Sie ist schwach antibiotisch aktiv. Milchsäure ist beispielsweise in Mengen von bis zu 10 Gew.-%, vorzugsweise 0,2 bis 5,0 Gew.-% in der Tensidzubereitung enthalten.

Weitere desinfizierende Inhaltsstoffe sind beispielsweise Wirkstoffe aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-Acetale sowie Formale, Benzamidine, Isothiazole und deren Derivate wie Isothiazoline und Isothiazolinone, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, Iodo-2-propynyl-butyl-carbamat, Iod, Iodophore und Peroxide. Hierunter bevorzugte Wirkstoffe werden vorzugsweise ausgewählt aus der Gruppe umfassend 1,3-Butandiol, Phenoxyethanol, 1,2-Propylenglykol, Glycerin, Undecylensäure, Zitronensäure, Milchsäure, Benzoeesäure, Salicylsäure, Thymol, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraazatetradecandiimidamid, quaternäre oberflächenaktive Verbindungen, Guanidine. Bevorzugte oberflächenaktive quaternäre Verbindungen enthalten eine Ammonium-, Sulfonium-, Phosphonium-, Jodonium- oder Arsoniumgruppe. Weiterhin können auch desinfizierende ätherische Öle eingesetzt werden, die gleichzeitig für eine Beduftung der viruziden Behandlungslösung sorgen. Besonders bevorzugte Wirkstoffe sind jedoch ausgewählt aus der Gruppe umfassend Salicylsäure, quaternäre Tenside, insbesondere Benzalkoniumchlorid, PeroxoVerbindungen, insbesondere Wasserstoffperoxid, Alkalimetallhypochlorit sowie Gemische derselben. Ein solcher weiterer desinfizierender Inhaltsstoff ist beispielsweise in einer Menge von 0,01 bis 1 Gew.-%, vorzugsweise 0,02 bis 0,8 Gew.-%, insbesondere 0,05 bis 0,5 Gew.-%, besonders bevorzugt 0,1 bis 0,3 Gew.-%, äußerst bevorzugt 0,2 Gew.-% in der Tensidzubereitung enthalten.

Flüssige erfindungsgemäße Tensidzubereitungen in Form von übliche Lösungsmittel enthaltenden Lösungen werden in der Regel durch einfaches Mischen der Inhaltsstoffe, die in Substanz oder als Lösung in einen automatischen Mischer gegeben werden können, hergestellt.

In einer weiteren bevorzugten Ausführungsform der Erfindung ist ein erfindungsgemäßes Verfahren dadurch gekennzeichnet, dass die das hydrolytische Enzym stabilisierende Komponente im wesentlichen frei von Borsäure ist oder einen Borgehalt von weniger als 50%, 45%, 40%, 35%, 30%, 25%, 20%, 15%, 10% oder 5% aufweist, bezogen auf die Gesamtmenge der das hydrolytische Enzym stabilisierenden Komponente. Ganz besonders bevorzugt ist die das hydrolytische Enzym stabilisierende Komponente borfrei.

In einer weiteren bevorzugten Ausführungsform der Erfindung ist ein erfindungsgemäßes Verfahren dadurch

gekennzeichnet, dass die das hydrolytische Enyzm stabilisierende Komponente einen reversiblen Inhibitor umfasst, der ausgewählt ist aus

a) Monosaccharidglycerat, insbesondere ein Aldoseglycerat, ein Halbacetal eines Aldoseglycerats, ein Ketoseglycerat oder ein Halbketal eines Ketoseglycerats, insbesondere wobei das Monosaccharid im Monosaccharidglycerat

a. ein Trioserest ist, insbesondere Glycerinaldehyd oder Dihydroxyaceton, oder
b. ein Tetroserest ist, insbesondere Erythrose, Threose oder Erythrulose, oder
c. ein Pentoserest ist, insbesondere Ribose, Arabinose, Xylose, Lyxose, Desoxyribose, Ribulose oder Xylulose, oder
d. ein Hexoserest ist, insbesondere Allose, Altrose, Glucose, Mannose, Gulose, Idose, Galaktose, Talose, Fucose, Rhamnose, Chinovose oder Fructose;

b) Oligoamino-biphenyl-oligocarbonsäure, insbesondere eine Diamino-biphenyl-dicarbonsäure, insbesondere 3,3-diamino [1,1-biphenyl]-2,4-dicarbonsäure;
c) Aminophthalsäure, insbesondere 4-Aminophthalsäure;
d) Phthaloylglutaminsäure;
e) mehrfach substituierte Benzolcarbonsäure, die an mindestens zwei Kohlenstoffatomen des Benzolrestes eine Carboxylgruppe aufweist, insbesondere eine mehrfach substituierte Benzolcarbonsäure, die eine Benzolcarbonsäure mit vier Carboxylgruppen am Benzolrest ist, insbesondere Pyromellitsäure;
f) Phenylalkyldicarbonsäure, insbesondere Phenylmalonsäure oder Benzylmalonsäure;
g) Polyol, insbesondere Glycerin, 1,2-Ethylenglykol, 1,2-Propylenglykol oder 1,3-Propylenglykol;
h) Antioxidans oder Glycerinsäure;
i) Calciumverbindung, Lactat oder ein Lactatderivat;
j) Borsäure;
k) Phenylboronsäure-Derivat mit der Strukturformel

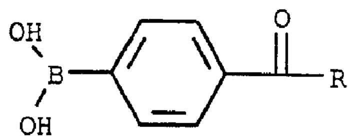

,

in der R für Wasserstoff, eine Hydroxyl-, eine C1-C6 Alkyl-, eine substituierte C1-C6 Alkyl-, eine C1-C6 Alkenyl oder eine substituierte C1-C6 Alkenyl-Gruppe steht, vorzugsweise 4-Formyl-phenylboronsäure (4-FPBA);
l) ein- oder mehrfach substituierte Phenylboronsäure, die an mindestens einem Kohlenstoffatom des Phenylrestes eine Hydroxylgruppe aufweist, insbesondere eine Phenylboronsäure, die an einem Kohlenstoffatom des Phenylrestes eine Hydroxylgruppe aufweist, insbesondere wobei die Reste an den weiteren Kohlenstoffatomen des Phenylrestes -H sind, insbesondere eine Hydroxyphenylboronsäure, vorzugsweise 3-Hydroxy-Phenyl-Boronsäure (3-HPBA).

Die das hydrolytische Enzym stabilisierende Komponente kann ein Monosaccharidglycerat als reversiblen Inhibitor umfassen. Hierunter wird eine Substanz verstanden, in der ein Monosaccharidrest über eine Acetalbindung an einen Glyceratrest kovalent gebunden ist. Ein Monosaccharidglycerat im Rahmen der vorliegenden Erfindung wird demnach durch nachstehende Formel (I) beschrieben:

$$\text{(I)}\quad \begin{array}{l} HOOC\text{-}CH\text{-}O\text{-}R \\ \qquad\quad | \\ \qquad\ CH_2OH \end{array},$$

wobei R ein Monosaccharidrest ist.

Ein Monosaccharid im Rahmen der vorliegenden Erfindung ist ein Produkt der partiellen Oxidation eines mehrwertigen Alkohols. Ein Monosaccharid besitzt eine Kette aus mindestens drei Kohlenstoffatomen als Grundgerüst und weist eine Carbonylgruppe sowie mindestens eine Hydroxygruppe auf. Nach der Anzahl der Kohlenstoffatome werden Triosen (3), Tetrosen (4), Pentosen (5), Hexosen (6), Heptosen (7) usw. unterschieden. Prinzipiell ist die Länge der Kohlenstoffkette unbegrenzt, vorzugsweise weist die Kohlenstoffkette zwischen drei und neun Kohlenstoffatome auf.

Das Monosaccharid kann ferner in offenkettiger Form (nichtcyclischer) oder cyclischer Form vorliegen. An einem der Kohlenstoffatome der offenkettigen Form liegt ein doppelt gebundenes Sauerstoff-Atom vor, so dass eine Carbonylgruppe vorhanden ist. Befindet sich diese Carbonylgruppe an einem Ende der Kohlenstoffkette, liegt eine Aldehydgruppe vor und das Monosaccharid ist eine Aldose. Befindet sich die Carbonylgruppe innerhalb der Kohlenstoffkette, liegt eine Ketogruppe vor und das Monosaccharid ist eine Ketose. Die cyclischen Monosaccharide sind von den entsprechenden Aldosen oder Ketosen abgeleitete Halbacetale oder Halbketale. Derartige Monosaccharide sind vorzugsweise Furanosen (Fünfringe) oder Pyranosen (Sechsringe) mit jeweils einem Sauerstoffatom im Ring, die jeweils $\alpha$- oder $\beta$-Konfiguration besitzen können.

Das Monosaccharid kann ferner in jeder möglichen stereoisomeren Form vorliegen. Auf Grund der räumlichen Anordnung der Hydroxylgruppen des Monosaccharids kann das Monosaccharid in D-oder L-Konfiguration vorliegen, wobei die Konfiguration in fachüblicher Art und Weise anhand der Fischer-Projektion des Monosaccharids ermittelt wird, bei der die C-C-Bindungen in gedachter (thermodynamisch ungünstigster) ekliptische Stellung senkrecht übereinander und auf der Papierebene ausgerollt dargestellt werden, und die Substituenten (hier Wasserstoffatome und Hydroxylgruppen) je nach Konfiguration rechts bzw. links aufgeführt werden, so dass sich eine eindeutige Konfiguration ergibt. Das am weitesten vom anomeren C-Atom entfernte chirale C-Atom bildet in Rechtsstellung die D-Konfiguration, in Linksstellung die L-Konfiguration.

Bevorzugte Monosaccharide entsprechen der allgemeine Summenformel: $C_nH_{2n}O_n$, wobei n eine Zahl zwischen drei und neun, bevorzugt zwischen vier und sechs, und besonders bevorzugt sechs ist.

Zu den Monosacchariden zählen ferner Derivate dieser Verbindungen, die nicht der vorstehenden allgemeinen Summenformel entsprechen. Solche Derivate weisen weitere chemische Modifikationen auf, insbesondere können sie einen oder mehrere Methyl-, Formyl-, Ethyl-, Acetyl-, t-Butyl-, Anisyl-, Benzyl-, Trifluroacetyl-, N-hydroxysuccinimid-, t-Butyloxycarbonyl-, Benzoyl-, 4-Methylbenzyl-, Thioanizyl-, Thiocresyl-, Benzyloxymethyl-, 4-Nitrophenyl-, Benzyloxycarbonyl-, 2-Nitrobenzoyl-, 2-Nitrophenylsulphenyl-, 4-Toluenesulphonyl-, Pentafluorophenyl-, Diphenylmethyl-, 2-Chlorobenzyloxycarbonyl-, 2,4,5-trichlorophenyl-, 2-bromobenzyloxycarbonyl-, 9-Fluorenylmethyloxycarbonyl-, Triphenylmethyl-, 2,2,5,7,8-pentamethyl-chroman-6-sulphonyl-Reste oder Kombinationen hiervon enthalten. Beispielsweise sind D-Glucuronsäure (6-Carboxy-D-glucose), D-Galacturonsäure (6-Carboxy-D-galactose), N-Acetyl-D-glucosamin (auch N-Acetylchitosamin), D-Glucosamin (auch Chitosamin), N-Acetyl-D-galactosamin (auch N-Acetylchondrosamin) oder D- und L-Fucose (6-Desoxy-D- und -L-galactose) Monosaccharide im Sinne der vorliegenden Erfindung.

Vorzugsweise ist das Monosaccharidglycerat ein Aldoseglycerat, ein Halbacetal eines Aldoseglycerats, ein Ketoseglycerat oder ein Halbketal eines Ketoseglycerats. Besonders bevorzugt ist das Monosaccharid im Monosaccharidglycerat

a. ein Trioserest, insbesondere Glycerinaldehyd oder Dihydroxyaceton, oder
b. ein Tetroserest, insbesondere Erythrose, Threose oder Erythrulose, oder
c. ein Pentoserest, insbesondere Ribose, Arabinose, Xylose, Lyxose, Desoxyribose, Ribulose oder Xylulose, oder
d. ein Hexoserest, insbesondere Allose, Altrose, Glucose, Mannose, Gulose, Idose, Galaktose, Talose, Fucose, Rhamnose, Chinovose oder Fructose.

Derartige Monosaccharidglycerate sind besonders wirksam als Verbindungen, die im Rahmen der vorliegenden Erfindung als das hydrolytische Enzym stabilisierende Komponente vorgesehen sind. In einer besonders bevorzugten Ausführungsform der Erfindung ist das Monosaccharidglycerat Glucosylglycerat. Ein besonders bevorzugtes Glucosylglycerat ist in nachfolgender Formel (II) angegeben:

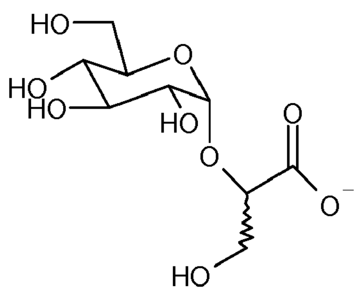

(II)

Die das hydrolytische Enzym stabilisierende Komponente kann eine Oligoamino-biphenyl-oligocarbonsäure als reversiblen Inhibitor umfassen, insbesondere eine Diamino-biphenyldicarbonsäure, insbesondere 3,3-diamino [1,1-biphenyl]-2,4-dicarbonsäure.

Eine Oligoamino-biphenyl-oligocarbonsäure im Rahmen der vorliegenden Erfindung wird durch nachstehende

Formel (III) beschrieben:

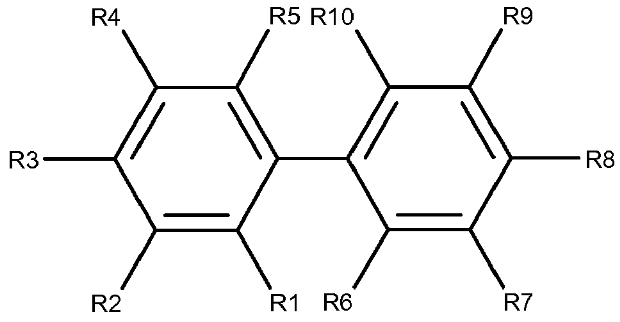

(III) ,

wobei R1, R2, R3, R4, R5, R6, R7, R8, R9 und R10 jeweils $-NH_2$, -COOH oder -H sind, wobei mindestens zwei der Reste R1 bis R10 - $NH_2$ sind und mindestens zwei der Reste R1 bis R10 -COOH sind und die weiteren Reste -H sind. Vorzugsweise befinden sich diejenigen Reste R1 bis R10, die - $NH_2$ sind, und diejenigen Reste R1 bis R10, die -COOH sind, an benachbarten Kohlenstoffatomen (C-Atomen), d.h. in ortho-Position zueinander.

Weiter bevorzugt sind genau zwei der Reste R1 bis R10 - $NH_2$ und zwei der Reste R1 bis R10 -COOH und die weiteren Reste -H. Eine derartige Ausführungsform der Erfindung ist folglich dadurch gekennzeichnet, dass die Oligoamino-biphenyl-oligocarbonsäure eine Diamino-biphenyl-dicarbonsäure ist. Besonders bevorzugt ist R1 -COOH, R2 $-NH_2$, R7 $-NH_2$, R8 -COOH, und R3, R4, R5, R6, R9, R10 jeweils -H. In einer derartigen besonders bevorzugten Ausführungsform der Erfindung ist die Oligoamino-biphenyl-oligocarbonsäure 3,3-diamino [1,1-biphenyl]-2,4-dicarbonsäure, die in nachfolgender Formel (IV) angegeben ist:

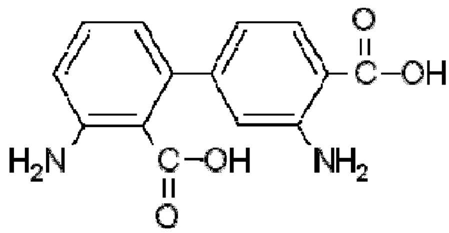

(IV)

Zu einer Oligoamino-biphenyl-oligocarbonsäure im Rahmen der Erfindung zählen ferner Derivate dieser Verbindungen. Solche Derivate weisen weitere chemische Modifikationen auf, insbesondere können sie an einer oder mehreren der Aminogruppen glykosyliert, oxidiert, N-methyliert, N-formyliert, N-acetyliert sein oder einen oder mehrere Methyl-, Amino-, Nitro-, Chloro-, Fluoro-, Bromo-, Hydroxyl-, Carboxyl-, Formyl-, Ethyl-, Acetyl-, t-Butyl-, Anisyl-, Benzyl-, Trifluroacetyl-, N-hydroxysuccinimid-, t-Butyloxycarbonyl-, Benzoyl-, 4-Methylbenzyl-, Thioanizyl-, Thiocresyl-, Benzyloxymethyl-, 4-Nitrophenyl-, Benzyloxycarbonyl-, 2-Nitrobenzoyl-, 2-Nitrophenylsulphenyl-, 4-Toluenesulphonyl-, Pentafluorophenyl-, Diphenylmethyl-, 2-Chlorobenzyloxycarbonyl-, 2,4,5-trichlorophenyl-, 2-bromobenzyloxycarbonyl-, 9-Fluorenylmethyloxycarbonyl-, Triphenylmethyl-, 2,2,5,7,8-pentamethyl-chroman-6-sulphonyl-Reste bzw. Gruppen oder Kombinationen hiervon enthalten.

Die das hydrolytische Enzym stabilisierende Komponente kann Aminophthalsäure als reversiblen Inhibitor umfassen, insbesondere 4-Aminophthalsäure.

Hierunter wird eine Benzoldicarbonsäure verstanden, die an mindestens einer weiteren Position des Phenylrestes einen $-NH_2$-Rest aufweist. Die beiden Carboxylgruppen der Benzoldicarbonsäure können sich in ortho-, meta- oder para-Position zueinander befinden. Bevorzugt befinden sie sich in ortho-Position zueinander. Der-$NH_2$-Rest befindet sich an einem derjenigen Kohlenstoffatome (C-Atome) des Phenylrestes, an dem keine Carboxylgruppe vorhanden ist. Die Reste an den verbleibenden Positionen des Phenylrestes sind vorzugsweise -H.

Eine bevorzugte derartige Verbindung wird durch nachstehende Formel (V) beschrieben:

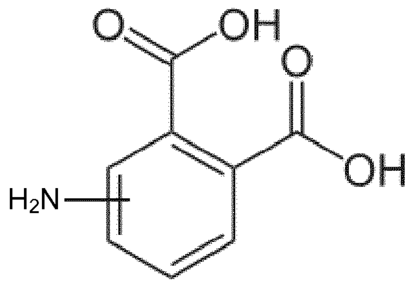

(V):

Darin befindet sich die $-NH_2$-Gruppe an dem Kohlenstoffatom (C-Atom) C3 oder C4 des Phenylrestes, besonders bevorzugt an C4 des Phenylrestes.

Zu einer Aminophthalsäure im Rahmen der Erfindung zählen ferner Derivate dieser Verbindung. Solche Derivate weisen weitere chemische Modifikationen auf, insbesondere können sie an der Aminogruppe glykosyliert, oxidiert, N-methyliert, N-formyliert oder N-acetyliert sein. Alternativ oder ergänzend können sie einen oder mehrere Methyl-, Amino-, Nitro-, Chloro-, Fluoro-, Bromo-, Hydroxyl-, Formyl-, Ethyl-, Acetyl-, t-Butyl-, Anisyl-, Benzyl-, Trifluroacetyl-, N-hydroxysuccinimid-, t-Butyloxycarbonyl-, Benzoyl-, 4-Methylbenzyl-, Thioanizyl-, Thiocresyl-, Benzyloxymethyl-, 4-Nitrophenyl-, Benzyloxycarbonyl-, 2-Nitrobenzoyl-, 2-Nitrophenylsulphenyl-, 4-Toluenesulphonyl-, Pentafluorophenyl-, Diphenylmethyl-, 2-Chlorobenzyloxycarbonyl-, 2,4,5-trichlorophenyl-, 2-bromobenzyloxycarbonyl-, 9-Fluorenylmethyloxycarbonyl-, Triphenylmethyl-, 2,2,5,7,8-pentamethyl-chroman-6-sulphonyl-Reste bzw. Gruppen oder Kombinationen hiervon enthalten.

Die das hydrolytische Enzym stabilisierende Komponente kann Phthaloylglutaminsäure als reversiblen Inhibitor umfassen.

Hierunter wird eine Substanz verstanden, die durch nachstehende Formel (VI) beschrieben wird (N-Phthaloyl-L-glutaminsäure):

(VI) 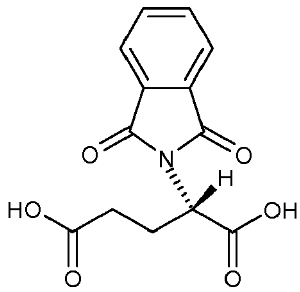

Alternativ oder ergänzend kann die das hydrolytische Enzym stabilisierende Komponente eine Phthaloylasparaginsäure als reversiblen Inhibitor umfassen. Hierunter wird eine Substanz verstanden, die durch nachstehende Formel (VII) beschrieben wird (N-Phthaloyl-L-asparaginsäure):

(VII) 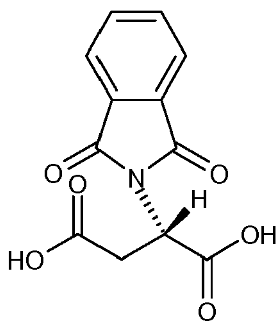

Zu einer Phthaloylglutaminsäure bzw. Phthaloylasparaginsäure im Rahmen der Erfindung zählen ferner Derivate dieser Verbindungen. Solche Derivate weisen weitere chemische Modifikationen auf, insbesondere können sie

glykosyliert sein oder am Phthaloyl-Rest einen oder mehrere Methyl-, Amino-, Nitro-, Chloro-, Fluoro-, Bromo-, Hydroxyl-, Carboxyl-, Formyl-, Ethyl-, Acetyl-, t-Butyl-, Anisyl-, Benzyl-, Trifluroacetyl-, N-hydroxysuccinimid-, t-Butyloxycarbonyl-, Benzoyl-, 4-Methylbenzyl-, Thioanizyl-, Thiocresyl-, Benzyloxymethyl-, 4-Nitrophenyl-, Benzyloxycarbonyl-, 2-Nitrobenzoyl-, 2-Nitrophenylsulphenyl-, 4-Toluenesulphonyl-, Pentafluorophenyl-, Diphenylmethyl-, 2-Chlorobenzyloxycarbonyl-, 2,4,5-trichlorophenyl-, 2-bromobenzyloxycarbonyl-, 9-Fluorenylmethyloxycarbonyl-, Triphenylmethyl-, 2,2,5,7,8-pentamethyl-chroman-6-sulphonyl-Reste bzw. Gruppen oder Kombinationen hiervon enthalten.

Die das hydrolytische Enzym stabilisierende Komponente kann eine mehrfach substituierte Benzolcarbonsäure als reversiblen Inhibitor umfassen.

Hierunter wird eine Benzolcarbonsäure verstanden, die an mindestens zwei Kohlenstoffatomen (C-Atomen) des Benzolrestes eine Carboxylgruppe (-COOH-Rest) aufweist. Bevorzugt weist die Benzolcarbonsäure drei, vier, fünf oder sechs Carboxylgruppen am Benzolrest auf. Weiter bevorzugt weist die Benzolcarbonsäure an denjenigen C-Atomen, die keine Carboxylgruppe tragen, einen Wasserstoffrest auf.

Die Carboxylgruppen der zweifach substituierten Benzolcarbonsäure können sich in ortho-, meta- oder para-Position zueinander befinden. Weitere Carboxylgruppen können sich an beliebigen zwischenliegenden Kohlenstoffatomen (C-Atomen) befinden.

Besonders bevorzugt handelt es sich bei der mehrfach substituierten Benzolcarbonsäure um eine Benzolcarbonsäure mit vier Carboxylgruppen am Benzolrest. Ganz besonders bevorzugt befinden sich die Carboxylgruppen an den C-Atomen C1, C2, C4 und C5 des Benzolrestes. Eine derartige Verbindung ist Pyromellitsäure. Sie weist Carboxylgruppen an den C-Atomen C1, C2, C4 und C5 des Benzolrestes und Wasserstoff an den C-Atomen C3 und C6 auf und stellt eine ganz besonders bevorzugte Ausgestaltung der das hydrolytische Enzym stabilisierenden Komponente dar. Sie ist in nachstehender Formel (VIII) angegeben:

(VIII) 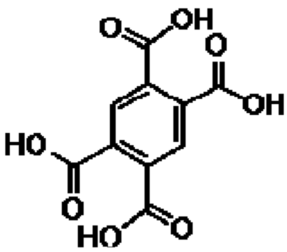

Zu einer mehrfach substituierten Benzolcarbonsäure im Rahmen der Erfindung zählen ferner Derivate dieser Verbindungen. Solche Derivate weisen weitere chemische Modifikationen auf, insbesondere können sie einen oder mehrere Methyl-, Amino-, Nitro-, Chloro-, Fluoro-, Bromo,-Hydroxyl-, Formyl-, Ethyl-, Acetyl-, t-Butyl-, Anisyl-, Benzyl-, Trifluroacetyl-, N-hydroxysuccinimid-, t-Butyloxycarbonyl-, Benzoyl-, 4-Methylbenzyl-, Thioanizyl-, Thiocresyl-, Benzyloxymethyl-, 4-Nitrophenyl-, Benzyloxycarbonyl-, 2-Nitrobenzoyl-, 2-Nitrophenylsulphenyl-, 4-Toluenesulphonyl-, Pentafluorophenyl-, Diphenylmethyl-, 2-Chlorobenzyloxycarbonyl-, 2,4,5-trichlorophenyl-, 2-bromobenzyloxycarbonyl-, 9-Fluorenylmethyloxycarbonyl-, Triphenylmethyl-, 2,2,5,7,8-pentamethyl-chroman-6-sulphonyl-Reste bzw. Gruppen oder Kombinationen hiervon enthalten.

Die das hydrolytische Enzym stabilisierende Komponente kann eine Phenylalkyldicarbonsäure als reversiblen Inhibitor umfassen, insbesondere Phenylmalonsäure.

Eine Phenylalkyldicarbonsäure im Rahmen der vorliegenden Erfindung wird durch nachstehende Formel (IX) beschrieben:

(IX) $C_6H_5$-$(CH_2)_n$-$CH(COOH)_2$,

wobei n eine ganze Zahl zwischen 0 und 14 ist und zunehmend bevorzugt zwischen 0 und 10, zwischen 0 und 5, zwischen 0 und 4, zwischen 0 und 3, zwischen 0 und 2, und ganz besonders bevorzugt 0 oder 1 ist. Besonders bevorzugte Ausgestaltungen der Erfindung sind folglich dadurch gekennzeichnet, dass die Phenylalkyldicarbonsäure Phenylmalonsäure oder Benzylmalonsäure ist. Phenylmalonsäure ist in nachstehender Formel (X), Benzylmalonsäure in der nachstehenden Formel (XI), angegeben:

(X): $C_6H_5CH(COOH)_2$

(XI):

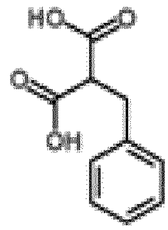

Zu einer Phenylalkyldicarbonsäure im Rahmen der Erfindung zählen ferner Derivate dieser Verbindung. Solche Derivate weisen weitere chemische Modifikationen auf, insbesondere können sie glykosyliert oder oxidiert sein oder am Phenylrest einen oder mehrere Methyl-, Amino-, Nitro-, Chloro-, Fluoro-, Bromo-, Hydroxyl-, Carboxyl-, Formyl-, Ethyl-, Acetyl-, t-Butyl-, Anisyl-, Benzyl-, Trifluroacetyl-, N-hydroxysuccinimid-, t-Butyloxycarbonyl-, Benzoyl-, 4-Methylbenzyl-, Thioanizyl-, Thiocresyl-, Benzyloxymethyl-, 4-Nitrophenyl-, Benzyloxycarbonyl-, 2-Nitrobenzoyl-, 2-Nitrophenylsulphenyl-, 4-Toluenesulphonyl-, Pentafluorophenyl-, Diphenylmethyl-, 2-Chlorobenzyloxycarbonyl-, 2,4,5-trichlorophenyl-, 2-bromobenzyloxycarbonyl-, 9-Fluorenylmethyloxycarbonyl-, Triphenylmethyl-, 2,2,5,7,8-pentamethyl-chroman-6-sulphonyl-Reste bzw. Gruppen oder Kombinationen hiervon enthalten.

Die das hydrolytische Enzym stabilisierende Komponente kann als reversiblen Inhibitor ferner ein Polyol umfassen, insbesondere Glycerin, 1,2-Ethylenglykol, 1,2-Propylenglykol oder 1,3-Propylenglykol. Die das hydrolytische Enzym stabilisierende Komponente kann als reversiblen Inhibitor ferner ein Antioxidans oder Glycerinsäure, eine Calciumverbindung, Lactat oder ein Lactatderivat umfassen. Die das hydrolytische Enzym stabilisierende Komponente kann ferner Borsäure als reversiblen Inhibitor umfassen.

Die das hydrolytische Enzym stabilisierende Komponente kann als reversiblen Inhibitor ferner ein Phenylboronsäure-Derivat mit der Strukturformel

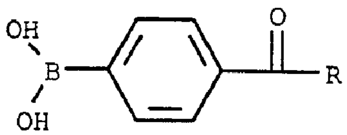

umfassen, in der R für Wasserstoff, eine Hydroxyl-, eine C1-C6 Alkyl-, eine substituierte C1-C6 Alkyl-, eine C1-C6 Alkenyl oder eine substituierte C1-C6 Alkenyl-Gruppe steht, vorzugsweise 4-Formyl-phenyl-boronsäure (4-FPBA).

Die das hydrolytische Enzym stabilisierende Komponente kann als reversiblen Inhibitor ferner eine ein- oder mehrfach substituierte Phenylboronsäure, die an mindestens einem Kohlenstoffatom des Phenylrestes eine Hydroxylgruppe aufweist, umfassen, insbesondere eine Phenylboronsäure, die an einem Kohlenstoffatom des Phenylrestes eine Hydroxylgruppe aufweist, insbesondere wobei die Reste an den weiteren Kohlenstoffatomen des Phenylrestes -H sind. Besonders bevorzugt handelt es sich um eine Hydroxyphenylboronsäure, ganz besonders bevorzugt um 3-Hydroxy-Phenyl-Boronsäure (3-HPBA). Sie wird durch nachstehende Formel (XII) beschrieben:

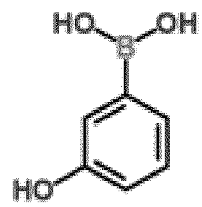

(XII):

Alle Verbindungen, die im Rahmen der vorliegenden Erfindung als das hydrolytische Enzym stabilisierende Komponente vorgesehen sind, können in allen protonierten oder deprotonierten Formen vorliegen. Ferner können alle derartigen Verbindungen, insbesondere deren deprotonierte Formen, mit Kationen vergesellschaftet sein. Bevorzugte Kationen sind diesbezüglich zweiwertige Kationen, insbesondere Ca-Ionen ($Ca^{2+}$), Mg-Ionen ($Mg^{2+}$) und Zn-Ionen ($Zn^{2+}$). Besonders bevorzugt sind Ca-Ionen ($Ca^{2+}$). Weiterhin können die Verbindungen in allen möglichen stereoisomeren Formen vorliegen.

Ein weiterer Gegenstand der Erfindung sind hydrolytische Enzyme, die durch ein erfindungsgemäßes Verfahren wie vorstehend beschrieben erhältlich sind. Bevorzugte hydrolytische Enzyme sind Proteasen und Amylasen, insbesondere Proteasen. Weiter bevorzugte hydrolytische Enzyme sind ausgewählt aus der Gruppe bestehend aus

a) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitution S166A aufweist;
b) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitution S166V aufweist;
c) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitution S166Y aufweist;
d) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitution S166G aufweist;
e) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitution A187D aufweist;
f) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitution F189R aufweist;
g) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitution Q191R aufweist;
h) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitutionen S166G und Q191R aufweist;
i) Protease mit einer Aminosäuresequenz, die zu SEQ ID NO. 3 über deren Gesamtlänge zu mindestens 90% und zunehmend bevorzugt zu mindestens 91%, 92%, 93%, 94%, 95%, 96%, 97%, 98%, 99% und 100% identisch ist und in der Zählung gemäß SEQ ID NO. 1 die Aminosäuresubstitutionen A187D und F189R aufweist.

Der Identitätswert, insbesondere der Identitätswert von 100%, bezieht sich hierbei auf die jeweilige Protease ohne die geforderte(n) Substitution(en), also auf das Grundgerüst (Grundsequenz) der Protease. In dieses jeweilige Grundgerüst sind dann die Substitutionen wie jeweils angegeben einzubringen. Beispielsweise ist eine Protease mit einer Aminosäuresequenz, die zu SEQ ID NO. 3 über deren Gesamtlänge zu 100% identisch ist und in der Zählung gemäß SEQ ID NO. 1 die Aminosäuresubstitution S166A aufweist, eine Protease gemäß SEQ ID NO. 3 mit der Aminosäuresubstitution S166A (in der Zählweise gemäß SEQ ID NO. 1). Entsprechendes gilt für die anderen Proteasen.
In einer weiteren bevorzugten Ausführungsform weisen die vorstehend genannten Proteasen nur die jeweils angegebene Aminosäuresubstitution beziehungsweise die jeweils angegebenen Aminosäuresubstitutionen auf.
In einer weiteren bevorzugten Ausführungsform ist das hydrolytische Enzym dadurch gekennzeichnet, dass es eine verminderte relative Aktivität im Vergleich mit der relativen Aktivität des Ausgangsenzyms gegenüber dem reversiblen Inhibitor, insbesondere gegenüber einem der reversiblen Inhibitoren wie vorstehend angegeben, aufweist. Vorzugsweise ist das hydrolytische Enzym eine Protease oder eine Amylaase, insbesondere eine Protease und ganz besonders bevorzugt eine Protease wie vorstehend beschrieben. Weitere bevorzugte Ausgestaltungen dieser Proteasen sind dadurch gekennzeichnet, dass die

a) Protease gemäß a) eine verminderte relative Aktivität im Vergleich mit der relativen Aktivität einer Protease gemäß SEQ ID NO. 3 gegenüber Borsäure, 4-Formyl-phenyl-boronsäure (4-FPBA) oder 3-Hydroxy-Phenyl-Boronsäure (3-HPBA), insbesondere 4-Formyl-phenyl-boronsäure (4-FPBA), aufweist;
b) Protease gemäß b) eine verminderte relative Aktivität im Vergleich mit der relativen Aktivität einer Protease gemäß SEQ ID NO. 3 gegenüber Borsäure, 4-Formyl-phenyl-boronsäure (4-FPBA) oder 3-Hydroxy-Phenyl-Boronsäure (3-HPBA), insbesondere 3-Hydroxy-Phenyl-Boronsäure (3-HPBA), aufweist;
c) Protease gemäß c) eine verminderte relative Aktivität im Vergleich mit der relativen Aktivität einer Protease gemäß SEQ ID NO. 3 gegenüber Borsäure oder 3-Hydroxy-Phenyl-Boronsäure (3-HPBA), insbesondere 3-Hydroxy-Phenyl-Boronsäure (3-HPBA), aufweist;
d) Protease gemäß d) eine verminderte relative Aktivität im Vergleich mit der relativen Aktivität einer Protease gemäß SEQ ID NO. 3 gegenüber Borsäure aufweist;

e) Protease gemäß e) eine verminderte relative Aktivität im Vergleich mit der relativen Aktivität einer Protease gemäß SEQ ID NO. 3 gegenüber Borsäure aufweist;
f) Protease gemäß f) eine verminderte relative Aktivität im Vergleich mit der relativen Aktivität einer Protease gemäß SEQ ID NO. 3 gegenüber Borsäure oder Benzylmalonsäure, insbesondere Borsäure, aufweist;
g) Protease gemäß g) eine verminderte relative Aktivität im Vergleich mit der relativen Aktivität einer Protease gemäß SEQ ID NO. 3 gegenüber Borsäure oder Benzylmalonsäure, insbesondere Benzylmalonsäure, aufweist;
h) Protease gemäß h) eine verminderte relative Aktivität im Vergleich mit der relativen Aktivität einer Protease gemäß SEQ ID NO. 3 gegenüber Borsäure oder Benzylmalonsäure, insbesondere Benzylmalonsäure, aufweist;
i) Protease gemäß i) eine verminderte relative Aktivität im Vergleich mit der relativen Aktivität einer Protease gemäß SEQ ID NO. 3 gegenüber Borsäure oder Benzylmalonsäure, insbesondere Benzylmalonsäure, aufweist.

Diesbezüglich erfolgt die Bestimmung der relativen Aktivitäten vorzugsweise wie in Beispiel 1 beschrieben.

Beispiele:

Beispiel 1: Anpassung einer Protease an vorgegebene Verbindungen, die die Protease inhibieren und somit stabilisieren

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

Als hydrolytisches Enzym (Ausgangsenzym) wurde eine Protease gemäß SEQ ID NO. 3 verwendet. Ferner wurden die nachfolgend angegebenen reversiblen Proteaseinhibitoren jeweils als die das hydrolytische Enzym stabilisierende Komponente bereitgestellt:

1. Borsäure
2. 4-Formyl-Phenyl-Boronsäure (4-FPBA)
3. 3-Hydroxy-Phenyl-Boronsäure (3-HPBA)
4. Benzylmalonsäure (BMA)

Die auf einem Plasmid vorliegende Nukleinsäuresequenz des hydrolytischen Enzyms wurde unter Verwendung von ortsgerichteter Mutagenese ("site directed mutagenesis") mittels spezifischer, angepasster Primer unter Verwendung einer Phusion HotStartPolymerase an den Positionen S166, A187, F189 oder Q191 in Zählung gemäß SEQ ID NO. 1 einzeln oder in Kombination wie in den nachstehenden Tabellen 1 und 2 angegeben modifiziert und die entstehenden Varianten in einen geeigneten Bacillus-Wirt transformiert. Mittels eines proteasesensitiven Mediums wurden aktive Klone selektiert und die DNA -Sequenz nach Plasmidisolierung zwecks Identifikation sequenziert. Die modifizierten Proteasen wurden in dem Bacillus-Wirt exprimiert und für die Aktivitätsbestimmung bereitgestellt.

Die Aktivitätsbestimmung erfolgte in einer flüssigen Tensidzubereitung, die wie folgt zusammengesetzt war (alle Angaben in Gewichts-Prozent): 0,3-0,5% Xanthan, 0,2-0,4% AntiSchaummittel, 6-7% Glycerin, 0,3-0,5% Ethanol, 4-7% FAEOS (Fettalkoholethersulfat), 24-28% nichtionische Tenside, 1-2% Natriumcitrat (Dihydrat), 2-4% Soda, 14-16% Kokosnuss-Fettsäuren, 0,5% HEDP (1-Hydroxyethan-(1,1-di-phosphonsäure)), 0-0,4% PVP (Polyvinylpyrrolidon), 0-0,05% optischer Aufheller, 0-0,001 % Farbstoff, Rest demineralisiertes Wasser.

Zu einer 50%igen flüssigen Zubereitung der flüssigen Tensidzubereitung wurden 0,92 mM AAPF gegeben. Enthalten war ferner die Menge des zu testenden reversiblen Inhibitors, die benötigt wird, um das Ausgangsenzym um 20-40% zu inhibieren. 20 ul der jeweiligen Protease (Ausgangsenzym sowie modifizierte Proteasevarianten) wurden hinzugegeben und die proteolytische Aktivität bestimmt über die Freisetzung des Chromophors para-Nitroanilin (pNA) aus dem Substrat suc-L-Ala-L-Ala-L-Pro-L-Phe-p-Nitroanilid (suc-AAPF-pNA). Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgte bei einer Temperatur von 25°C, bei pH 8,6 und einer Wellenlänge von 410 nm.

Die gemessene Aktivität wurde mit der Aktivität eines Referenzansatzes der gleichen Proteaseprobe ohne Inhibitor verglichen und die jeweiligen relativen Aktivitäten der modifizierten Proteasen und des Ausgangsenzyms bestimmt. Ein stärkerer Rückgang der relativen Aktivität einer Variante bei dem jeweiligen Inhibitor als beim Ausgangsenzym zeigt eine bessere Inhibierung. Die Ergebnisse sind in den nachstehenden Tabellen 1 und 2 angegeben (jeweils angegeben sind die relativen Aktivitäten).

Tabelle 1:

| Enzym | Ohne Inhibitor | Borsäure | 4-FPBA | 3-HPBA |
|---|---|---|---|---|
| S166 | 100% | 83% | 75% | 83% |
| S166A | 100% | 76% | 61% | 83% |
| S166V | 100% | 59% | 71% | 53% |
| S166Y | 100% | 68% | 100% | 58% |

Tabelle 2:

| Enzym | Ohne Inhibitor | Borsäure | BMA |
|---|---|---|---|
| S166 | 100% | 79% | 81% |
| A187D | 100% | 47% | 79% |
| F189R | 100% | 53% | 80% |
| Q191R | 100% | 76% | 63% |
| S166G | 100% | 65% | 80% |
| S166G/Q191R | 100% | 57% | 35% |
| A187D/F189R | 100% | 37% | 43% |

Es wird deutlich, dass die nach Durchführung eines erfindungsgemäßen Verfahrens erhaltenen Proteasen besser an die das hydrolytische Enzym stabilisierende Komponente, insbesondere den reversiblen Inhibitor, angepasst sind und folglich von dieser verbessert inhibiert bzw. stabilisiert werden. Eine verbesserte Inhibierung der nach Durchführung eines erfindungsgemäßen Verfahrens erhaltenen Proteasen wurde für die in Tabelle 1 angegebenen Varianten auch mit Benzylmalonsäure (BMA) als reversiblem Inhibitor erreicht.

Beispiel 2: Inhibierungsmessung im biochemischen Test

Die Varianten S166V und S166G/Q191R sowie das Ausgangsenzym gemäß SEQ ID NO. 3 wurden in einem geeigneten Expressionssystem exprimiert, mittels Ionenaustauschchromatographie weiter gereinigt und für die Aktivitätsbestimmungen in 0,1 M Tris/ HCl-Puffer pH 8,6, enthaltend 0,1% Brij35, bereitgestellt. Zu den Ansätzen wurden steigende Mengen des reversiblen Inhibitors 3-HPBA gegeben bzw. BMA (vgl. Tabellen 3 und 4) und die proteolytische Restaktivität der jeweiligen Protease über die Freisetzung des Chromophors para-Nitroanilin (pNA) aus dem Substrat suc-L-Ala-L-Ala-L-Pro-L-Phe-p-Nitroanilid (suc-AAPF-pNA) wie vorstehend beschrieben bestimmt. Die erhaltenen Aktivitäten sind in den Tabellen 3 und 4 angegeben.

Tabelle 3:

| Enzym | Ohne Inhibitor | 3-HPBA (in mM) | | | | |
|---|---|---|---|---|---|---|
| | | 0,05 | 0,1 | 0,5 | 1 | 1,5 |
| Ausgangsenzym | 100% | 93% | 89% | 81% | 65% | 56% |
| S166V | 100% | 84% | 87% | 65% | 51% | 40% |

Tabelle 4:

| Enzym | Ohne Inhibitor | BMA (in Gew.-%) | | | | | | | |
|---|---|---|---|---|---|---|---|---|---|
| | | 0,05 | 0,5 | 1,0 | 1,7 | 2,5 | 3,3 | 4,0 | 5,0 |
| Ausgangsenzym | 100% | 96% | 95% | 93% | 94% | 74% | 73% | 78% | 64% |
| S166G/Q191R | 100% | 88% | 55% | 42% | 35% | 38% | 32% | 36% | 32% |

Es wird deutlich, dass die jeweilige Variante eine stärkere Inhibierbarkeit durch den reversiblen Inhibitor als das Ausgangsenzym zeigt. Die Durchführung eines erfindungsgemäßen Verfahrens führte folglich zum Erhalt einer Variante, die besser an einen vorgegeben reversiblen Inhibitor, hier 3-HPBA bzw. BMA, angepasst ist.

Beispiel 3: Bestimmung der Lagerstabilität in einer flüssigen Tensidzubereitung

Die Varianten S166V und S166G/Q191R sowie das Ausgangsenzym gemäß SEQ ID NO. 3 wurden konzentrationsgleich zu Reaktionsansätzen gegeben, die eine Tensidzubereitung wie in Beispiel 1 beschrieben sowie einen reversiblen Inhibitor in Konzentrationen wie in Tabelle 5 angegeben enthielten. Die Ansätze wurden bei 30°C gelagert und die verbleibende proteolytische Restaktivität der Protease über die Freisetzung des Chromophors para-Nitroanilin (pNA) aus dem Substrat suc-L-Ala-L-Ala-L-Pro-L-Phe-p-Nitroanilid (suc-AAPF-pNA) wie vorstehend beschrieben in regelmäßigen zeitlichen Abständen bestimmt. Die Zugabe der Inhibitoren führte zu den in Tabelle 5 angegeben Erhöhungsfaktoren der Halbwertszeiten (Halbwertszeit mit Inhibitor dividiert durch Halbwertszeit ohne Inhibitor).

Tabelle 5:

| Enzym | Borsäure (in Gew.-%) | | 3-HPBA (in Gew.-%) | | BMA (in Gew.-%) |
|---|---|---|---|---|---|
| | 1 | 0,25 | 0,02 | 0,005 | 1 |
| Ausgangsenzym | 26 | 4 | 14 | 5 | 3 |
| S166V | 43 | 6 | 34 | 9 | n.b. |
| S166G/Q191R | n.b. | n.b. | n.b. | n.b. | 5 |
| (n.b.: nicht bestimmt) | | | | | |

Es wird deutlich, dass die jeweilige Variante auch in einer Tensidzubereitung eine stärkere Inhibierbarkeit durch den reversiblen Inhibitor als das Ausgangsenzym zeigt. Die Durchführung eines erfindungsgemäßen Verfahrens führte folglich zum Erhalt einer Variante, die auch im Kontext einer Tensidzubereitung besser an einen vorgegeben reversiblen Inhibitor, hier neben Borsäure insbesondere 3-HPBA und BMA, angepasst ist.

**Patentansprüche**

**1.** Verfahren zur Anpassung eines hydrolytischen Enzyms an eine das hydrolytische Enzym stabilisierende Komponente umfassend die Verfahrensschritte

a) Bereitstellen eines hydrolytischen Enzyms (Ausgangsenzym) und einer das hydrolytische Enzym stabilisierenden Komponente, die einen reversiblen Inhibitor des hydrolytischen Enzyms umfasst;
b) Verändern der Aminosäuresequenz des hydrolytischen Enzyms an mindestens einer Position durch Substitution, Deletion oder Insertion;
c) Bestimmen der relativen Aktivität des hydrolytischen Enzyms aus Schritt b) und der relativen Aktivität des Ausgangsenzyms in einer flüssigen Zubereitung;
d) Auswählen desjenigen hydrolytischen Enzyms, das eine verminderte relative Aktivität aufweist im Vergleich

mit der relativen Aktivität des Ausgangsenzyms, wobei das durch das Verfahren erhältliche hydrolytische Enzym eine Protease ist, deren Aminosäuresequenz zu SEQ ID NO. 3 über deren Gesamtlänge zu mindestens 90% identisch ist, und in der Zählung gemäß SEQ ID NO. 1 Aminosäuresubstitution(en) ausgewählt aus S166A, S166V, S166Y, S166G, A187D, F189R, Q191R, S166G + Q191R, oder A187D + F189R aufweist.

**2.** Verfahren nach Anspruch 1, wobei die Protease eine verminderte relative Aktivität im Vergleich mit der relativen Aktivität einer Protease gemäß SEQ ID NO. 3 gegenüber Borsäure, Benzylmalonsäure 4-Formyl-phenyl-boronsäure (4-FPBA) und/oder 3-Hydroxy-Phenyl-Boronsäure (3-HPBA) aufweist.

**3.** Verfahren nach Anspruch 1 und 2, **dadurch gekennzeichnet, dass** der reversible Inhibitor bezüglich des hydrolytischen Enzyms in Verfahrensschritt a) eine Inhibitionskonstante ($K_i$) von 0,01 bis 500 mM, vorzugsweise von 0,01 bis 100 mM, besonders bevorzugt von 0,01 bis 1 mM oder von 10 bis 100 mM aufweist.

**4.** Verfahren nach Anspruch 1 bis 3, **dadurch gekennzeichnet, dass** die Protease an einer Position verändert wird, die der Position 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 167, 168, 169, 170, 171, 172, 173, 174, 175, 176, 177, 178, 179, 180, 181, 182, 183, 184, 185, 186, 188, 190, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274 oder 275 in SEQ ID NO. 1, zugeordnet in einem Alignment mit SEQ ID NO. 1, entspricht.

**5.** Verfahren nach einem der Ansprüche 1 bis 4, **dadurch gekennzeichnet, dass** die das hydrolytische Enzym stabilisierende Komponente in einer Menge von 0,000001 bis 10 Gew.-% in der flüssigen Zubereitung enthalten ist, und/oder das hydrolytische Enzym in einer Menge von 1 x $10^{-8}$ bis 5 Gewichts-Prozent in der flüssigen Zubereitung enthalten ist bezogen auf den Gesamtproteingehalt des hydrolytischen Enzyms.

**6.** Verfahren nach einem der Ansprüche 1 bis 5, **dadurch gekennzeichnet, dass** die flüssige Zubereitung eine Tensidzubereitung ist.

**7.** Verfahren nach einem der Ansprüche 1 bis 6, **dadurch gekennzeichnet, dass** die das hydrolytische Enzym stabilisierende Komponente frei von Borsäure ist oder einen Borgehalt von weniger als 50 Gew.-% aufweist oder borfrei ist.

**8.** Verfahren nach einem der Ansprüche 1 bis 7, **dadurch gekennzeichnet, dass** die das hydrolytische Enzym stabilisierende Komponente eine Verbindung umfasst, die ausgewählt ist aus

a) Monosaccharidglycerat, insbesondere ein Aldoseglycerat, ein Halbacetal eines Aldoseglycerats, ein Ketoseglycerat oder ein Halbketal eines Ketoseglycerats, insbesondere wobei das Monosaccharid im Monosaccharidglycerat

a. ein Trioserest ist, insbesondere Glycerinaldehyd oder Dihydroxyaceton, oder
b. ein Tetroserest ist, insbesondere Erythrose, Threose oder Erythrulose, oder
c. ein Pentoserest ist, insbesondere Ribose, Arabinose, Xylose, Lyxose, Desoxyribose, Ribulose oder Xylulose, oder
d. ein Hexoserest ist, insbesondere Allose, Altrose, Glucose, Mannose, Gulose, Idose, Galaktose, Talose, Fucose, Rhamnose, Chinovose oder Fructose;

b) Oligoamino-biphenyl-oligocarbonsäure, insbesondere eine Diamino-biphenyl-dicarbonsäure, insbesondere 3,3-diamino [1,1-biphenyl]-2,4-dicarbonsäure;
c) Aminophthalsäure, insbesondere 4-Aminophthalsäure;
d) Phthaloylglutaminsäure;
e) mehrfach substituierte Benzolcarbonsäure, die an mindestens zwei Kohlenstoffatomen des Benzolrestes

eine Carboxylgruppe aufweist, insbesondere eine mehrfach substituierte Benzolcarbonsäure, die eine Benzolcarbonsäure mit vier Carboxylgruppen am Benzolrest ist, insbesondere Pyromellitsäure;
f) Phenylalkyldicarbonsäure, insbesondere Phenylmalonsäure oder Benzylmalonsäure;
g) Polyol, insbesondere Glycerin, 1,2-Ethylenglykol, 1,2-Propylenglykol oder 1,3-Propylenglykol
h) Antioxidans oder Glycerinsäure;
i) Calciumverbindung, Lactat oder ein Lactatderivat;
j) Borsäure;
k) Phenylboronsäure-Derivat mit der Strukturformel

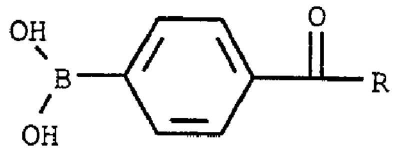

,

in der R für Wasserstoff, eine Hydroxyl-, eine C1-C6 Alkyl-, eine substituierte C1-C6 Alkyl-, eine C1-C6 Alkenyl oder eine substituierte C1-C6 Alkenyl-Gruppe steht, vorzugsweise 4-Formyl-phenyl-boronsäure (4-FPBA);
l) ein- oder mehrfach substituierte Phenylboronsäure, die an mindestens einem Kohlenstoffatom des Phenylrestes eine Hydroxylgruppe aufweist, insbesondere eine Phenylboronsäure, die an einem Kohlenstoffatom des Phenylrestes eine Hydroxylgruppe aufweist, insbesondere wobei die Reste an den weiteren Kohlenstoffatomen des Phenylrestes -H sind, insbesondere eine Hydroxyphenylboronsäure, vorzugsweise 3-Hydroxy-Phenyl-Boronsäure (3-HPBA).

Europäisches Patentamt
European Patent Office
Office européen des brevets

# EUROPÄISCHER RECHERCHENBERICHT

Nummer der Anmeldung
EP 16 16 4786

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 98/23732 A2 (GENENCOR INT [US]; JONES J BRYAN [CA] GENENCOR INT [US]; JONES J BRYAN) 4. Juni 1998 (1998-06-04)<br>* Abbildungen 1,2; Beispiel 4 *<br>----- | 1-8 | INV.<br>C11D3/386<br>C12N9/54 |
| A,D | WO 96/21716 A1 (NOVO NORDISK AS [DK]; LYKKE MADS [DK]; SIMONSEN OLE [DK]) 18. Juli 1996 (1996-07-18)<br>* Ansprüche 1,6,8; Beispiel 3 *<br>----- | 1-8 | |
| A | KELLER T H ET AL: "Probing the specificity of the S1 binding site of subtilisin Carlsberg with boronic acids", BIOCHEMICAL AND BIOPHYSICAL RESEARCH COMMUNICATIONS, ACADEMIC PRESS INC. ORLANDO, FL, US, Bd. 176, Nr. 1, 15. April 1991 (1991-04-15), Seiten 401-405, XP024772684, ISSN: 0006-291X, DOI: 10.1016/0006-291X(91)90938-4 [gefunden am 1991-04-15]<br>* Zusammenfassung; Tabelle 1 *<br>----- | 1-8 | RECHERCHIERTE SACHGEBIETE (IPC)<br>C11D<br>C12N |
| A | WO 2005/118793 A2 (HENKEL KGAA [DE]; WIELAND SUSANNE [DE]; WEBER ANGRIT [DE]; BECKERS ANG) 15. Dezember 2005 (2005-12-15)<br>* Zusammenfassung *<br>-----<br>-/-- | 1-8 | |

Der vorliegende Recherchenbericht wurde für alle Patentansprüche erstellt

1

| Recherchenort | Abschlußdatum der Recherche | Prüfer |
|---|---|---|
| München | 21. Juni 2016 | Griesinger, Irina |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 16 16 4786

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | SEUFER-WASSERTHAL P ET AL: "Probing the specificity of the S1 binding site of subtilisin Carlsberg with boronic acids", BIOORGANIC & MEDICINAL CHEMISTRY, PERGAMON, GB, Bd. 2, Nr. 1, 1. Januar 1994 (1994-01-01), Seiten 35-48, XP026615430, ISSN: 0968-0896, DOI: 10.1016/S0968-0896(00)82200-2 [gefunden am 1994-01-01] * Seite 39, rechte Spalte * ----- | 1-8 | |
| Y | WO 2008/002472 A2 (DANISCO US INC GENENCOR DIV [US]; AEHLE WOLFGANG [US]; ESTELL DAVID A) 3. Januar 2008 (2008-01-03) * Ansprüche 1,16 * ----- | 1-8 | |
| Y | WO 2004/099401 A1 (NOVOZYMES AS [DK]; MINNING STEFAN [DK]; KNOETZEL JUERGEN CARSTEN FRANZ) 18. November 2004 (2004-11-18) * Sequenz 4 * ----- | 1-8 | RECHERCHIERTE SACHGEBIETE (IPC) |
| Y | WO 98/55634 A1 (PROCTER & GAMBLE [US]; GENENCOR INT [US]; RAI SAROJ [US]; CORREA PAUL) 10. Dezember 1998 (1998-12-10) * Sequenz 9 * ----- | 1-8 | |
| Y | US 2004/091474 A1 (RAVEN NEIL DAVID HAMMOND [GB] ET AL) 13. Mai 2004 (2004-05-13) * Sequenz 12 * ----- | 1-8 | |

1 Der vorliegende Recherchenbericht wurde für alle Patentansprüche erstellt

| Recherchenort | Abschlußdatum der Recherche | Prüfer |
|---|---|---|
| München | 21. Juni 2016 | Griesinger, Irina |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 16 16 4786

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-06-2016

| Im Recherchenbericht angeführtes Patentdokument | Datum der Veröffentlichung | Mitglied(er) der Patentfamilie | Datum der Veröffentlichung |
|---|---|---|---|
| WO 9823732 A2 | 04-06-1998 | AT 349514 T | 15-01-2007 |
| | | AU 733082 B2 | 03-05-2001 |
| | | AU 7417398 A | 22-06-1998 |
| | | CA 2273079 A1 | 04-06-1998 |
| | | CA 2763810 A1 | 04-06-1998 |
| | | CN 1246146 A | 01-03-2000 |
| | | DE 69737148 T2 | 04-10-2007 |
| | | DK 0942962 T3 | 07-05-2007 |
| | | EP 0942962 A2 | 22-09-1999 |
| | | ES 2277364 T3 | 01-07-2007 |
| | | JP 2002506342 A | 26-02-2002 |
| | | NZ 336434 A | 30-03-2001 |
| | | US 6277617 B1 | 21-08-2001 |
| | | WO 9823732 A2 | 04-06-1998 |
| WO 9621716 A1 | 18-07-1996 | AR 000649 A1 | 10-07-1997 |
| | | AU 4328396 A | 31-07-1996 |
| | | BR 9606684 A | 09-06-1998 |
| | | CA 2208705 A1 | 18-07-1996 |
| | | CN 1168155 A | 17-12-1997 |
| | | EP 0802968 A1 | 29-10-1997 |
| | | FI 972896 A | 08-07-1997 |
| | | JP H10511855 A | 17-11-1998 |
| | | WO 9621716 A1 | 18-07-1996 |
| WO 2005118793 A2 | 15-12-2005 | DE 102004027091 A1 | 29-12-2005 |
| | | EP 1789546 A2 | 30-05-2007 |
| | | WO 2005118793 A2 | 15-12-2005 |
| WO 2008002472 A2 | 03-01-2008 | CA 2654269 A1 | 03-12-2008 |
| | | CN 101473036 A | 01-07-2009 |
| | | DK 2032698 T3 | 03-09-2012 |
| | | EP 2032698 A2 | 11-03-2009 |
| | | ES 2388753 T3 | 18-10-2012 |
| | | HK 1128934 A1 | 01-03-2013 |
| | | JP 5785686 B2 | 30-09-2015 |
| | | JP 2009540862 A | 26-11-2009 |
| | | KR 20090023408 A | 04-03-2009 |
| | | PL 2032698 T3 | 30-11-2012 |
| | | PT 2032698 E | 24-09-2012 |
| | | US 2008004186 A1 | 03-01-2008 |
| | | US 2011082048 A1 | 07-04-2011 |
| | | US 2014187440 A1 | 03-07-2014 |
| | | WO 2008002472 A2 | 03-01-2008 |
| WO 2004099401 A1 | 18-11-2004 | AT 407202 T | 15-09-2008 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 16 16 4786

In diesem Anhang sind die Mitglieder der Patentfamilien der im obengenannten europäischen Recherchenbericht angeführten Patentdokumente angegeben. Die Angaben über die Familienmitglieder entsprechen dem Stand der Datei des Europäischen Patentamts am Diese Angaben dienen nur zur Unterrichtung und erfolgen ohne Gewähr.

21-06-2016

| Im Recherchenbericht angeführtes Patentdokument | Datum der Veröffentlichung | Mitglied(er) der Patentfamilie | Datum der Veröffentlichung |
|---|---|---|---|
| | | AU 2004236308 A1 | 18-11-2004 |
| | | BR PI0409992 A | 09-05-2006 |
| | | CA 2526341 A1 | 18-11-2004 |
| | | CN 1784490 A | 07-06-2006 |
| | | CN 102766545 A | 07-11-2012 |
| | | EP 1623029 A1 | 08-02-2006 |
| | | JP 4819670 B2 | 24-11-2011 |
| | | JP 2006524998 A | 09-11-2006 |
| | | US 2007015677 A1 | 18-01-2007 |
| | | WO 2004099401 A1 | 18-11-2004 |
| WO 9855634 A1 | 10-12-1998 | AT 338823 T | 15-09-2006 |
| | | CA 2293695 A1 | 10-12-1998 |
| | | DE 69835814 T2 | 18-01-2007 |
| | | DK 0985042 T3 | 15-01-2007 |
| | | EP 0985042 A1 | 15-03-2000 |
| | | ES 2273417 T3 | 01-05-2007 |
| | | JP 4031055 B2 | 09-01-2008 |
| | | JP 2002502256 A | 22-01-2002 |
| | | PT 985042 E | 31-01-2007 |
| | | US 6369011 B1 | 09-04-2002 |
| | | WO 9855634 A1 | 10-12-1998 |
| US 2004091474 A1 | 13-05-2004 | US 2004091474 A1 | 13-05-2004 |
| | | US 2008178306 A1 | 24-07-2008 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9621716 A1
- WO 9641859 A1
- WO 9219707 A1
- EP 478050 A1
- EP 511456 A1
- WO 08086916 A
- WO 07131656 A
- WO 9102792 A
- WO 08007319 A
- WO 9318140 A
- WO 0144452 A
- GB 1243784 A
- WO 9634946 A
- WO 02029024 A
- WO 03057246 A
- WO 03002711 A
- WO 03054177 A
- WO 07079938 A
- WO 200060060 A


### In der Beschreibung aufgeführte Nicht-Patentliteratur


- **LEONOR MICHAELIS ; MAUD MENTEN.** Die Kinetik der Invertinwirkung. *Biochem. Z.,* 1913, vol. 49, 333-369
- **GLEICHUNG 2 ; CHENG Y. ; PRUSOFF W.H.** *Biochem.Pharmacol.,* 1973, vol. 22, 3099-3108
- *Tenside,* 1970, vol. 7, 125-132
- **ALTSCHUL, S.F. ; GISH, W. ; MILLER, W. ; MYERS, E.W. ; LIPMAN, D.J.** Basic local alignment search tool. *J. Mol. Biol.,* 1990, vol. 215, 403-410
- **ALTSCHUL, STEPHAN F. ; THOMAS L. MADDEN ; ALEJANDRO A. SCHAFFER ; JINGHUI ZHANG ; HHENG ZHANG ; WEBB MILLER ; DAVID J. LIPMAN.** Gapped BLAST and PSI-BLAST: a new generation of protein database search programs. *Nucleic Acids Res.,* 1997, vol. 25, 3389-3402
- **CHENNA et al.** Multiple sequence alignment with the Clustal series of programs. *Nucleic Acid Research,* 2003, vol. 31, 3497-3500
- **NOTREDAME et al.** T-Coffee: A novel method for multiple sequence alignments. *J. Mol. Biol.,* 2000, vol. 302, 205-217
- **A. G. GORNALL ; C. S. BARDAWILL ; M.M. DAVID.** *J. Biol. Chem.,* 1948, vol. 177, 751-766